# EUROPEAN PATENT APPLICATION

(11) **EP 2 591 782 A1**
(43) Date of publication of application: **15.05.2013**
(21) Application number: 11188782.4
(22) Date of filing: 11.11.2011
(51) Int. Cl.: A61K 31/437, C07D 471/04, A61P 19/08, A61P 19/10

(54) **Nadph oxidase 4 inhibitors and use thereof**

(71) Applicant: Johann Wolfgang Goethe-Universität, 60325 Frankfurt am Main (DE); GenKyoTex SA, 1228 Plan-les-Ouates (CH)
(72) Inventor: Brandes, Ralf, 60596 FRANKFURT (DE); Schröder, Katrin, 99441 GROSSSCHWABHAUSEN (DE); Page, Patrick, F-74160 SAINT- JULIEN-EN-GENEVOIS (FR); Laleu, Benoît, F-74160 COLLONGES-SOUS-SALEVE (FR); Gaggini, Francesca, CH-1205 GENEVA (CH)
(74) Representative: reuteler & cie SA

(57) **Abstract**

The present invention is related to Nox4 inhibitors, pharmaceutical composition thereof and to their use for the treatment and/or prevention of osteoporosis or an osteoclastogenesis dysfunction, in particular osteoporotic and pre-osteoporotic osteoclastogenesis dysfunction.

## Description

### Field of the Invention

The present invention relates to agents useful in the treatment and/or prevention of osteoporosis and/or an osteoclastogenesis dysfunction such as an osteoporotic or pre-osteoporotic osteoclastogenesis dysfunction.

### Background of the Invention

Bone is a dynamic organ undergoing permanent remodeling depending on the needs of the organism even in adulthood. Bone formation is mediated by osteoblasts, while osteoclasts, which derive from the myeloid linage, absorb bone. *In vivo* as well as in culture model, osteoclastogenesis requires the differentiation factors "receptor activator of nuclear factor kappa B ligand (RANKL) of the TNF- superfamily and macrophage colony stimulating factor (M-CSF). M-CSF induces the expression of RANK, the receptor of RANKL on myeloid cells, whereas RANK ligand binding initiates the osteoclastogenic program and the induction of osteoclast genes such as tartrate-resistant acid phosphatase (TRAP) and cathepsin K. Even mature osteoclasts are activated by RANKL, which promotes their survival and induces structural changes leading to the resorption of bone. Osteoclastogenesis can further be promoted by cytokines like TGFβ. TGFβ has been linked to oxidative stress as it increases the formation of reactive oxygen species (ROS) and induces the expression of NADPH oxidase Nox4 *(*Sturrock et al., 2006, Am. J. Physiol Lung Cell Mol. Physiol., 290:L661-L673*).*

NADPH oxidases (NOX) are proteins that transfer electrons across biological membranes. In general, the electron acceptor is oxygen and the product of the electron transfer reaction is superoxide. The biological function of NOX enzymes is therefore the generation of reactive oxygen species (ROS) from oxygen. Reactive oxygen species (ROS) are oxygen-derived small molecules, including oxygen radicals (super-oxide anion [^{•}O₂⁻], hydroxyl [HO^{•}], peroxyl [ROO^{•}], alkoxyl [RO^{•}] and hydroperoxyl [HOO^{•}]) and certain non-radicals that are either oxidizing agents and/or are easily converted into radicals like hydrogen peroxide (H₂O₂). Nitrogen-containing oxidizing agents, such as nitric oxide are also called reactive nitrogen species (RNS). ROS generation is generally a cascade of reactions that starts with the production of superoxide or hydrogen peroxide. Superoxide also dismutates to hydrogen peroxide either spontaneously, particularly at low pH or catalyzed by superoxide dismutase. Other elements in the cascade of ROS generation include the reaction of superoxide with nitric oxide to form peroxynitrite, the peroxidase-catalyzed formation of hypochlorous acid from hydrogen peroxide, and the iron-catalyzed Fenton reaction leading to the generation of hydroxyl radical.

ROS avidly interact with a large number of molecules including other small inorganic molecules as well as DNA, proteins, lipids, carbohydrates and nucleic acids. This initial reaction may generate a second radical, thus multiplying the potential damage. ROS are involved not only in cellular damage and killing of pathogens, but also in a large number of reversible regulatory processes in virtually all cells and tissues. However, despite the importance of ROS in the regulation of fundamental physiological processes, ROS production can also irreversibly destroy or alter the function of the target molecule. Consequently, ROS have been increasingly identified as major contributors to damage in biological organisms, so-called "oxidative stress".

Although the only known function of Nox proteins is the production of reactive oxygen species (ROS), Nox4 differs from other NADPH oxidases in that it produces hydrogen peroxidase rather than superoxide and that the activity of the constitutively active enzyme is controlled by the expression level and not signaling pathways leading to acute activation *(*Takac et al., 2011, J. Biol. Chem., 286:13304-13313*;* Helmcke et al., 2009, Antioxid Redox. Signal., 11:1279-1287*).* NADPH oxidase Nox4 is ubiquitously expressed in differentiated cells and induction of Nox4 have been linked to the process of differentiation in mesenchymal cells like cardiac myocytes, adipocytes and smooth muscle cells.

Oxidative stress, by an increased action of osteoclasts, has been implicated in increased bone resorption and osteoporosis and osteoclastogenesis in cell culture models *(*Lee et al., 2005, Blood, 106:852-859*;* Sasaki et al., 2009, J. Med Invest, 56:33-41*).* However, although it has been shown that NADPH oxidases are upregulated during osteoclastogenesis and that inhibition of a component of all Nox proteins, p22phox prevents osteoclastogenesis (*Sasaki et al., 2009, above*), the role of each of the catalytically active Nox isoforms was unclear or controversial. Sasaki et al., 2009, Free Radical Biology & Medicine, 47, 189-199 provided evidence that Nox1 and Nox2 should be inhibited to limit osteoclastogenesis and that Nox4 inhibition was inefficient in blocking ROS production and osteoclast formation and *Lee et al., 2005 above* concluded that Nox1 rather than Nox2 or Nox4 were important for RANKL-induced ROS production in bone marrow monocyte-macrophage lineage cells.

Therefore, it would be highly desirable to develop new active agents for the treatment of osteoporosis focusing on their action on osteoclasts, bone resorption, and osteoclastogenesis.

### Summary of the Invention

The present invention is directed towards the unexpected findings that genetic Nox4 deletion leads to both a lower expression of the osteoclast marker protein osteoclast-associated receptor (OSCAR) and to the higher expression of the osteoblast marker protein osteopontin in bones and as consequence of this that Nox4 controls bone mass by regulation of osteoclastogenesis. In particular, the present invention is directed towards the unexpected findings that *in vivo* inhibition of Nox4 is able to prevent bone loss.

The present invention is directed to Nox4 inhibitors useful in the treatment and/or prophylaxis of osteoporosis, in particular osteoporotic or pre-osteoporotic osteoclastogenesis dysfunction. Notably, the invention is related to new molecules useful in the inhibition or reduction of Nox4 activity.

A first aspect of the invention provides a Nox4 inhibitor for the treatment and/or prophylaxis of osteoporosis.

A second aspect of the invention provides a use of one or more Nox4 inhibitor for the preparation of a pharmaceutical composition for the treatment and/or prophylaxis of osteoporosis.

A third aspect of the invention provides new Nox4 inhibitors, as well as tautomers, geometrical isomers, optically active forms, and pharmaceutically acceptable salts thereof.

A fourth aspect of the invention relates to new Nox4 inhibitors, as well as tautomers, geometrical isomers, optically active forms, and pharmaceutically acceptable salts thereof for use as a medicament.

A fifth aspect of the invention relates to a pharmaceutical composition containing at least one Nox4 inhibitor according to the invention, as well as tautomers, geometrical isomers, optically active forms and pharmaceutically acceptable salts thereof and a pharmaceutically acceptable carrier, diluent or excipient thereof.

A sixth aspect of the invention relates to a pharmaceutical composition containing at least one Nox4 inhibitor combined with at least one co-agent useful in the treatment or prevention of osteoporosis, and at least one pharmaceutically acceptable carrier.

A seventh aspect of the invention relates to a method for treating a patient suffering from osteoporosis comprising administering an effective amount of one or more Nox4 inhibitor, in a patient in need thereof.

An eighth aspect of the invention relates to a method for decreasing ostoeclastogenesis in bones of a patient comprising administering an effective amount of one or more Nox4 inhibitor or a pharmaceutical formulation thereof in a patient in need thereof.

A ninth aspect of the invention relates to a use of a Nox4 inhibitor for the preparation of a pharmaceutical formulation for the prevention and/or the treatment of osteoporosis.

A tenth aspect of the invention relates to a use of a Nox4 inhibitor for the preparation of a pharmaceutical formulation for the prevention and/or the treatment of osteoporotic osteoclastogenesis and pre-osteopororic osteoclastogenesis.

An eleventh aspect of the invention relates to a method of identifying inhibitors of ostoeclastogenesis.

Other features and advantages of the invention will be apparent from the following detailed description.

### Description of the figures

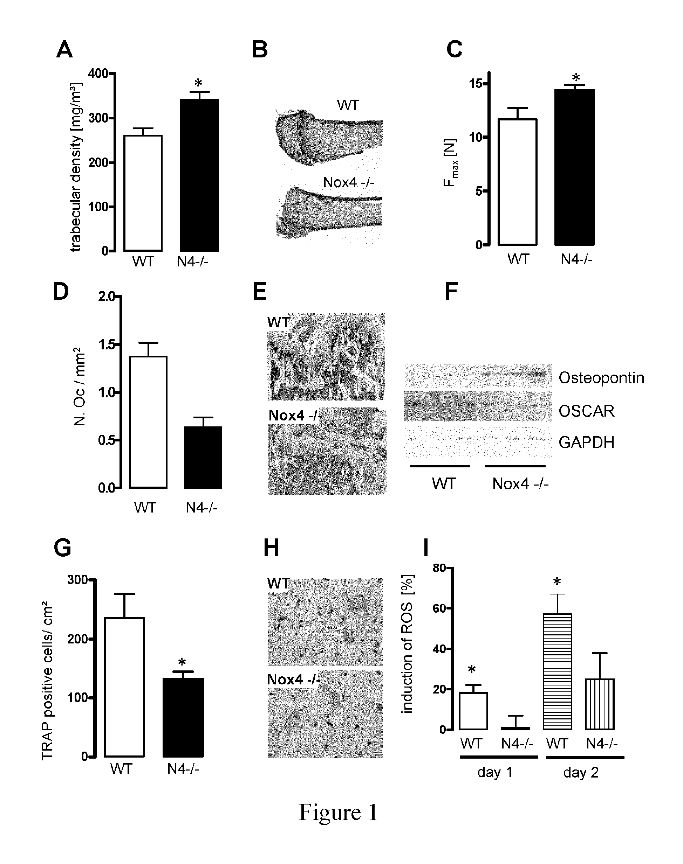

**Figure 1** shows the effect of genetic knockout of Nox4 on bone density, osteoclast number, differentiation and ROS formation in mice. **A:** Trabecular bone mineral density as measured in Example 2; **B:** Picture of undecalcified sections of distal femur stained with von Kossa technique as described in *von Kossa, 1901, Beit. Path. Anat., 29:163;* **C:** Ultimate force (Fmax) as measured in Example 2; **D:** Number of TRAP positive osteoclasts (N.Oc) counted in histomorphometric analysis as measured in Example 3 and **E:** representative pictures of TRAP-stained sections counter-stained with Mayer's hemalaun as described in Mukherjee et al., 2008, J. Clin. Invest., 118: 491-504 using protocol 387A-1KT; Sigma-Aldrich (www.sigma-aldrich.com); **F:** Western blot analysis from femur lysates for the osteoclast marker protein osteoclast-associated receptor (OSCAR), the osteoblast marker osteopontin and the loading control GADPH as described in Shen et al., 2005, J. Biol. Chem., 280, 40589-98*;* **G, H:** Number (G) and representative picture (H) of osteoclasts differentiated *ex vivo* from bone marrow cells as identified by TRAP staining as described in Example 3; **I:** ROS percentages in predifferentiated spleenocytes as measured in Example 3; n≥3; **p<0.05.*

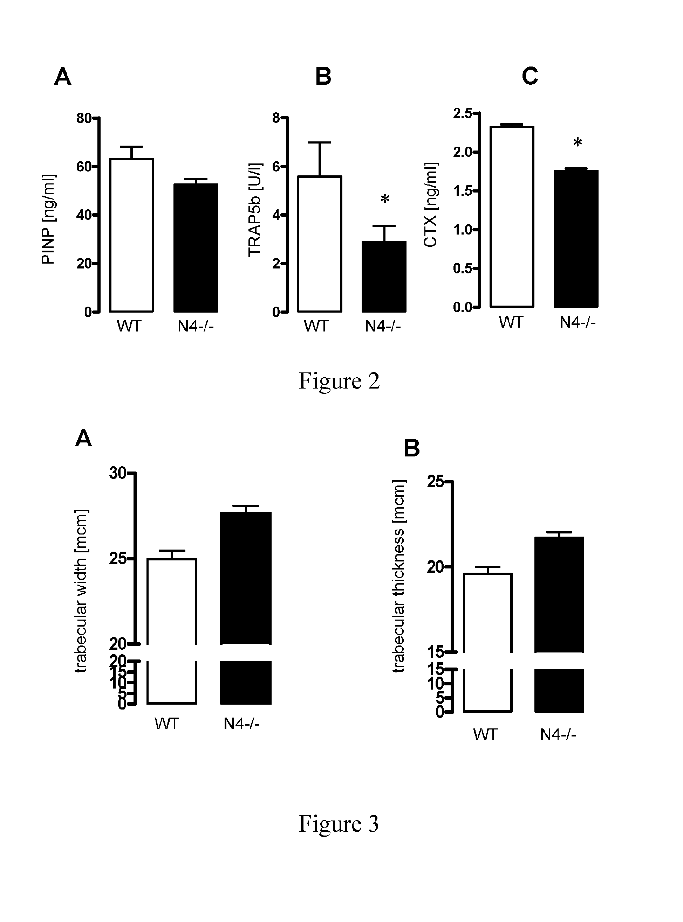

**Figure 2** shows the serum concentrations of a bone formation marker (procollagen1 N-terminal peptide, PINP) **(A)** compared to those of markers of bone resorption (TRAP 5b) **(B)** and carboxy-terminal collagen crosslinks (CTX) (C) measured as described in Example 3 in wild type and Nox4-/- mice.; n≥3; **p<0.05.*
**Figure 3** shows the histomorphometry of a distal femur measured as described in Example 2 from wildtype and Nox4-/- mice for **(A)** trabecular width and **(B)** thickness; n≥3; **p*<0.05.

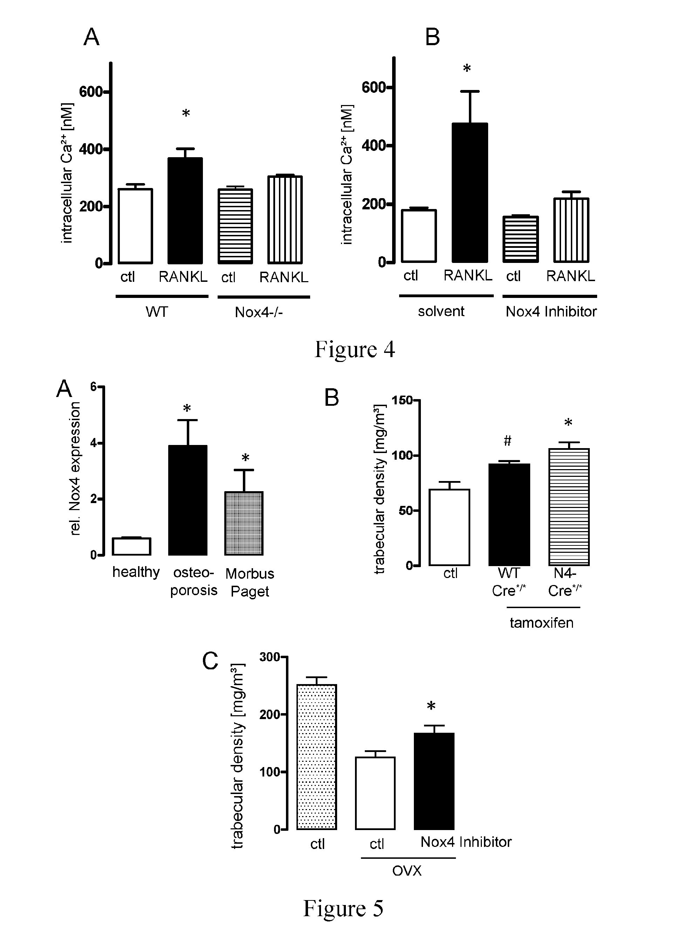

**Figure 4** shows the role of Nox4 in RANKL induced signaling and effect of a Nox4 inhibitor thereon by measurement of intracellular Ca²⁺ by Fura2 fluorescence in bone marrow-derived mononuclear cells primed with M-CSF pre-treated with (RANKL) or without RANKL (ctl) for 30 hours as described in Example 4. **A:** comparison of cells from wildtype and Nox4-/- mice; **B:** Effect of a Nox4 inhibitor (Compound (1) or solvent on calcium concentrations in wildtype cells; n≥3; **p<0.05.*
**Figure 5** shows the role of Nox4 for bone loss *in vivo* as described in Example 5. **A:** Statistical analyses of the intensity of Nox4 staining in human bone material from healthy subjects, and patients with osteoporosis or Morbus Paget n=3-4; **p*<0.05; **B,C:** Trabecular bone density of the distal femur 6 weeks after ovariectomy. Comparison of WT animals (no label), WT animals treated with tamoxifen (WT) and Nox4-Flox-Flox-ERT2-Cre+/0 mice treated with tamoxifen (N4-Cre*/*). n=8-12; *p<0.05 **(C);** Comparison of WT animals treated with solvent (ctl) or a Nox4 inhibitor n=11-14 for 6 weeks **(D).**

Statistical analysis: All values are mean ± SEM. Statistical analysis was performed by ANOVA followed by LSD post hoc testing. A p-value of less than 0.05 was considered statistically significant.WT: wild type mice; N4-/-: Nox4 knockout mice.

### Detailed Description of the invention

The following paragraphs provide definitions of the various chemical moieties that make up the compounds according to the invention and are intended to apply uniformly through-out the specification and claims, unless an otherwise expressly set out definition provides a broader definition.

"NOX4 inhibitor" as used herein refers to any substances that are able to totally or partially inhibit, block, attenuate, or interfere with NOX4, or with any pathway elicited, either directly or indirectly, by NOX4. Thus, the term "inhibitors" is intended to include, but is not limited to, molecules which inhibits completely or partially the activity of NADPH oxidase 4. According to a particular embodiment, Nox4 inhibitors have a major Nox inhibitory activity component compared to other Nox proteins, for example to Nox2.

For example, NOX4 inhibitors include substances which prevent or decrease differentiation of osteoclasts or decrease osteoclast population which are at the origin of the bone degeneration and, thus, which prevent, decrease or abolish osteoclastogenesis dysfunction. For example, NOX4 inhibitors include small molecules, peptides, peptidomimetics, chimeric proteins, natural or unnatural proteins, nucleic acid derived polymers (such as DNA and RNA aptamers, siRNAs, shRNAs, PNAs, or LNAs), fusion proteins with NOX4 antagonizing activities, antibody antagonists such as neutralizing anti-NOX4 antibodies, or gene therapy vectors driving the expression of such NOX4 antagonists.

The term "siRNA" refers to small interfering RNA which are double stranded RNA (about 19-23 nucleotides) able to knock down or silence a targeted mRNA from a target gene. Artificial siRNAs can be either chemically synthesized as oligonucleotides or cloned into a plasmid or a virus vector (adenovirus, retrovirus or lentivirus) as short hairpin RNAs to generate a transient or stable transfection in any type of cells *(*Martin et al., 2007, Ann. Rev. Genomics Hum. Genet., 8:81-108*;* Huang et al., 2008, Expert. Opin. Ther. Targets, 12(5), 637-645*).*

The term "efficacy" of a treatment or method according to the invention can be measured based on changes in the course of disease or condition in response to a use or a method according to the invention. For example, the efficacy of a treatment or method according to the invention can be measured by a reduction of bone loss, and/or an increase of bone mineral density (as measured by tomography or densitometry or dual energy x-ray absorptiometry and changes in plasma markers of bone turn-over such as collagene fragments and/or a decreased risk of osteoporotic facture in osteoporotic patients or by a reduction of in patients at risk of developing osteoporosis, in particular osteoclastogenesis dysfunction such as in post-menopausal women, in individual presenting eating disorders, or excessive use of medications such as oral corticosteroids (i.e. hydrocortisone) or anti-estrogens.

The term "effective amount" as used herein refers to an amount of at least one NOX4 inhibitor or a pharmaceutical formulation thereof according to the invention that elicits a detectable reduction of osteoclast differentiation and/or osteoclast counts or of osteoclastogenesis.

The term "osteoclastogenesis dysfunction" as used herein refers to any condition related with increased bone turn over or bone resorption of secondary cause such a Paget's bone loss resulting from immobilization, osteolytic bone metastasis and bone tumours. The term "osteoclastogenesis dysfunction" as used herein refers to any condition related with increased bone resorption of secondary cause such a Paget's disease, bone loss resulting from immobilization, osteolytic bone metastasis and bone tumours

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e., arresting its development; or relieving the disease, i.e., causing regression of the disease and/or its symptoms or conditions.

The term "subject" as used herein refers to mammals. For examples, mammals contemplated by the present invention include human, primates, domesticated animals such as cattle, sheep, pigs, horses and the like.

The term "pharmaceutically acceptable salts or complexes" refers to salts or complexes of the below-specified compounds of the invention. Examples of such salts include, but are not restricted, to base addition salts formed by reaction of compounds of the invention with organic or inorganic bases such as hydroxide, carbonate or bicarbonate of a metal cation such as those selected in the group consisting of alkali metals (sodium, potassium or lithium), alkaline earth metals (e.g. calcium or magnesium), or with an organic primary, secondary or tertiary alkyl amine. Other examples of such salts include, but are not restricted, to acid addition salts formed by reaction of compounds of the invention with organic or inorganic acids such as hydrochloric acid, oxalic acid or the like.

"Pharmaceutically active derivative" refers to any compound that upon administration to the recipient, is capable of providing directly or indirectly, the activity disclosed herein. The term "indirectly" also encompasses prodrugs which may be converted to the active form of the drug via endogenous enzymes or metabolism. The prodrug is a derivative of the compound according to the invention and presenting NADPH oxidase 4 inhibiting activity that has a chemically or metabolically decomposable group, and a compound that may be converted into a pharmaceutically active compound in vivo by solvolysis under physiological conditions. The invention further encompasses any tautomers of the compounds according to the invention.

### Nox4 inhibitors according to the invention

In one embodiment, the invention provides a NOX4 inhibitor presenting an inhibitory constant (Ki) for Nox4 inhibitory activity ranging from 10 nM or lower to 500 nM in functional assay of ROS production.

In another particular embodiment, is provided a NOX4 inhibitor is a siRNA.

In another particular embodiment, is provided a NOX4 inhibitor is an anti-NOX4 antibody.

In another particular embodiment, is provided a NOX4 inhibitor selected from the following group:
4-(2-fluoro-4-methoxyphenyl)-2-(2-methoxyphenyl)-5-(pyridin-3-ylmethyl)-1H-pyrazolo[4,3-c]pyridine-3,6(2H,5H)-dione;
2-(2-chlorophenyl)-4-(4-methoxyphenyl)-5-(pyrazin-2-ylmethyl)-1H-pyrazolo[4,3-c] pyridine-3,6(2H,5H)-dione;
4-(4-chlorophenyl)-2-(2-methoxyphenyl)-5-(pyrazin-2-ylmethyl)-1H-pyrazolo[4,3-c] pyridine-3,6(2H,5H)-dione;
2-(2-chlorophenyl)-4-(2-fluoro-4-methoxyphenyl)-5-[(1-methyl-1H-pyrazol-3-yl) methyl]-1H-pyrazolo[4,3-c]pyridine-3,6(2H,5H)-dione;
4-(2-fluoro-5-methoxyphenyl)-2-(2-methoxyphenyl)-5-(pyridin-3-ylmethyl)-1H-pyrazolo [4,3-c]pyridine-3,6(2H,5H)-dione;
2-(2-chlorophenyl)-5-[(2-methoxypyridin-4-yl)methyl]-4-methyl-1H-pyrazolo[4,3-c] pyridine-3,6(2H,5H)-dione;
2-(2-methoxyphenyl)-4-methyl-5-(pyridin-3-ylmethyl)-1H-pyrazolo[4,3-c]pyridine-3,6(2H,5H)-dione;
4-(4-chloro-2-fluorophenyl)-2-(2-methoxyphenyl)-5-(pyridin-3-ylmethyl)-1H-pyrazolo [4,3-c] pyridine-3,6(2H,5H)-dione;
4-(5-chloro-2-fluorophenyl)-2-(2-chlorophenyl)-5-(pyridin-3-ylmethyl)-1H-pyrazolo [4,3-c]pyridine-3,6(2H,5H)-dione;
2-(2-chlorophenyl)-5-[(6-methoxypyridin-3-yl)methyl]-4-methyl-1H-pyrazolo[4,3-c] pyridine-3,6 (2H,5H)-dione;
4-(4-chloro-2-fluorophenyl)-2-(2-chlorophenyl)-5-(pyridin-3-ylmethyl)-1H-pyrazolo [4,3-c]pyridine-3,6(2H,5H)-dione;
4-(5-chloro-2-fluorophenyl)-2-(2-chlorophenyl)-5-(pyridin-4-ylmethyl)-1H-pyrazolo [4,3-c]pyridine-3,6(2H,5H)-dione;
4-(2-fluoro-5-methoxyphenyl)-2-(2-methoxyphenyl)-5-[(1-methyl-1H-pyrazol-3-yl) methyl]-1H-pyrazolo[4,3-c]pyridine-3,6(2H,5H)-dione;
4-(5-chloro-2-fluorophenyl)-2-(2-methoxyphenyl)-5-(pyridin-3-ylmethyl)-1H-pyrazolo [4,3-c] pyridine-3,6(2H,5H)-dione;
2-(2-chlorophenyl)-4-methyl-5-(pyridin-3-ylmethyl)-1H-pyrazolo[4,3-c]pyridine-3,6 (2H,5H)-dione;
2-(2-chlorophenyl)-4-(4-chlorophenyl)-5-(pyrazin-2-ylmethyl)-1H-pyrazolo[4,3-c] pyridine-3,6 (2H,5H)-dione;
2-(2-chlorophenyl)-4-(2-fluorophenyl)-5-(pyridin-3-ylmethyl)-1H-pyrazolo[4,3-c] pyridine-3,6(2H,5H)-dione;
2-(2-chlorophenyl)-4-(4-chlorophenyl)-5-(pyridin-4-ylmethyl)-1H-pyrazolo[4,3-c] pyridine-3,6(2H,5H)-dione;
4-(4-chloro-2-fluorophenyl)-2-(2-chlorophenyl)-5-(pyridin-4-ylmethyl)-1H-pyrazolo [4,3-c]pyridine-3,6(2H,5H)-dione;
2-(2-methoxyphenyl)-4-(3-methoxyphenyl)-5-[(1-methyl-1H-pyrazol-3-yl)methyl]-1H-pyrazolo [4,3-c]pyridine-3,6(2H,5H)-dione;
2-(2-chlorophenyl)-4-(2-fluoro-4-methoxyphenyl)-5-(pyridin-3-ylmethyl)-1H-pyrazolo [4,3-c]pyridine-3,6(2H,5H)-dione;
4-(2-fluoro-4-methoxyphenyl)-2-(2-methoxyphenyl)-5-[(1-methyl-1H-pyrazol-3-yl) methyl]-1H-pyrazolo[4,3-c]pyridine-3,6(2H,5H)-dione;
2-(2-methoxyphenyl)-4-(4-methoxyphenyl)-5-[(1-methyl-1H-pyrazol-3-yl)methyl]-1H-pyrazolo [4,3-c]pyridine-3,6(2H,5H)-dione;
2-(2-methoxyphenyl)-4-(3-methoxyphenyl)-5-(pyridin-3-ylmethyl)-1H-pyrazolo[4,3-c] pyridine-3,6(2H,5H)-dione;
2-(2-chlorophenyl)-4-(4-chlorophenyl)-5-(pyridin-3-ylmethyl)-1H-pyrazolo[4,3-c] pyridine-3,6(2H,5H)-dione;
4-(4-chloro-2-fluorophenyl)-2-(2-chlorophenyl)-5-[(2-methoxypyridin-4-yl)methyl]-1H-pyrazolo[4,3-c]pyridine-3,6(2H,5H)-dione;
2-(2-chlorophenyl)-4-(2-fluoro-4-methoxyphenyl)-5-(pyridin-4-ylmethyl)-1H-pyrazolo [4,3-c]pyridine-3,6(2H,5H)-dione;
2-(2-chlorophenyl)-4-(2,6-difluorophenyl)-5-(pyridin-4-ylmethyl)-1H-pyrazolo[4,3-c] pyridine-3,6(2H,5H)-dione;
2-(2-chlorophenyl)-4-(2-fluorophenyl)-5-(pyridin-4-ylmethyl)-1H-pyrazolo[4,3-c] pyridine-3,6(2H,5H)-dione;
2-(2-chlorophenyl)-4-methyl-5-[(1-methyl-1H-pyrazol-3-yl)methyl]-1H-pyrazolo[4,3-c] pyridine-3,6(2H,5H)-dione;
4-(3-chloro-2-fluorophenyl)-2-(2-chlorophenyl)-5-(pyridin-4-ylmethyl)-1H-pyrazolo [4,3-c]pyridine-3,6(2H,5H)-dione;
2-(2-chlorophenyl)-5-methyl-4-[3-(methylamino)phenyl]-1H-pyrazolo[4,3-c]pyridine-3,6(2H,5H)-dione;
2-(2-methoxyphenyl)-4-(4-methoxyphenyl)-5-(pyridin-3-ylmethyl)-1H-pyrazolo[4,3-c] pyridine-3,6(2H,5H)-dione;
2-(2-chlorophenyl)-4-(2-fluorophenyl)-5-(pyridin-2-ylmethyl)-1H-pyrazolo[4,3-c] pyridine-3,6(2H,5H)-dione;
2-(2-chlorophenyl)-4-(2,5-difluorophenyl)-5-(pyridin-4-ylmethyl)-1H-pyrazolo[4,3-c] pyridine-3,6(2H,5H)-dione;
2-(2-chlorophenyl)-4-(4-chlorophenyl)-5-(1,3-thiazol-2-ylmethyl)-1H-pyrazolo[4,3-c]pyridine-3,6(2H,5H)-dione;
2-(2-chlorophenyl)-4-[3-(dimethylamino)phenyl]-5-[(1-methyl-1H-pyrazol-3-yl) methyl]-1H-pyrazolo[4,3-c]pyridine-3,6(2H,5H)-dione;
4-[3-(dimethylamino)phenyl]-2-(2-methoxyphenyl)-5-methyl-1H-pyrazolo[4,3-c] pyridine-3,6(2H,5H)-dione;
2-(2-chlorophenyl)-4-(3,5-dichlorophenyl)-5-(pyridin-4-ylmethyl)-1H-pyrazolo[4,3-c] pyridine-3,6(2H,5H)-dione;
4-(3-chloro-2-fluorophenyl)-2-(2-chlorophenyl)-5-(pyridin-3-ylmethyl)-1H-pyrazolo [4,3-c]pyridine-3,6(2H,5H)-dione;
2-(2-chlorophenyl)-4-[3-(dimethylamino)phenyl]-5-(pyridin-3-ylmethyl)-1H-pyrazolo [4,3-c]pyridine-3,6(2H,5H)-dione;
2-(2-chlorophenyl)-4-(2,6-difluorophenyl)-5-(pyridin-3-ylmethyl)-1H-pyrazolo[4,3-c] pyridine-3,6(2H,5H)-dione;
4-(2-fluoro-5-methoxyphenyl)-2-(2-methoxyphenyl)-5-(pyrazin-2-ylmethyl)-1H-pyrazolo[4,3-c]pyridine-3,6(2H,5H)-dione; and
2-(2-chlorophenyl)-4-(2,5-difluorophenyl)-5-(pyridin-3-ylmethyl)-1H-pyrazolo[4,3-c] pyridine-3,6(2H,5H)-dione.

In another particular embodiment, is provided a NOX4 inhibitor according of the invention for use as a medicament.

### Compositions

The invention provides pharmaceutical or therapeutic agents as compositions and methods for treating a patient, preferably a mammalian patient, and most preferably a human patient who is suffering from osteoporosis, in particular osteoclastogenesis dysfunction.

Pharmaceutical compositions of the invention can contain one or more Nox 4 inhibitor in any form described herein. Compositions of this invention may further comprise one or more pharmaceutically acceptable additional ingredient(s), such as alum, stabilizers, antimicrobial agents, buffers, coloring agents, flavoring agents, adjuvants, and the like. The compounds of the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed. Compositions according to the invention are preferably injectable.

Compositions of this invention may also be liquid formulations, including, but not limited to, aqueous or oily suspensions, solutions, emulsions, syrups, and elixirs. Liquid forms suitable for oral administration may include a suitable aqueous or non-aqueous vehicle with buffers, suspending and dispensing agents, colorants, flavors and the like. The compositions may also be formulated as a dry product for reconstitution with water or other suitable vehicle before use. Such liquid preparations may contain additives, including, but not limited to, suspending agents, emulsifying agents, non-aqueous vehicles and preservatives. Suspending agents include, but are not limited to, sorbitol syrup, methyl cellulose, glucose/sugar syrup, gelatin, hydroxyethylcellulose, carboxymethyl cellulose, aluminum stearate gel, and hydrogenated edible fats. Emulsifying agents include, but are not limited to, lecithin, sorbitan monooleate, and acacia. Non aqueous vehicles include, but are not limited to, edible oils, almond oil, fractionated coconut oil, oily esters, propylene glycol, and ethyl alcohol. Preservatives include, but are not limited to, methyl or propyl p-hydroxybenzoate and sorbic acid. Further materials as well as processing techniques and the like are set out in Part 5 of Remington's Pharmaceutical Sciences, 21st Edition, 2005, University of the Sciences in Philadelphia, Lippincott Williams & Wilkins*,* which is incorporated herein by reference. Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate. Tablets may be coated according to methods well known in the art.

Injectable compositions are typically based upon injectable sterile saline or phosphate-buffered saline or other injectable carriers known in the art.

Compositions of this invention may also be formulated for parenteral administration, including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.

Compositions of this invention may also be formulated as a depot preparation, which may be administered by implantation or by intramuscular injection. The compositions may be formulated with suitable polymeric or hydrophobic materials (as an emulsion in an acceptable oil, for example), ion exchange resins, or as sparingly soluble derivatives (as a sparingly soluble salt, for example).

The compounds of this invention can also be administered in sustained release forms or from sustained release drug delivery systems. A description of representative sustained release materials can also be found in the incorporated materials in *Remington's Pharmaceutical Sciences.*

### Mode of administration

Compositions of this invention may be administered in any manner, including, but not limited to, orally, parenterally, sublingually, via buccal administration, or combinations thereof. Parenteral administration includes, but is not limited to, intravenous, intraarterial, intra-peritoneal, subcutaneous, intramuscular, intra-thecal, and intra-articular. The compositions of this invention may also be administered in the form of an implant, which allows slow release of the compositions as well as a slow controlled i.v. infusion. In a particular embodiment, one or more Nox4 inhibitor is administered orally.

The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

### Combination

According to one embodiment of the invention, a Nox4 inhibitors and pharmaceutical formulations thereof can be administered alone or in combination with a co-agent useful in the prevention and/or treatment of osteoporosis or an osteoclastogeneis dysfunction, like bisphosphonate, estrogen or vitamin D.

The invention encompasses the administration of a Nox4 inhibitor or a pharmaceutical formulation thereof, wherein the Nox4 inhibitor or the pharmaceutical formulation thereof is administered to an individual prior to, simultaneously or sequentially with other therapeutic regimens or co-agents useful in the treatment of osteoporosis (e.g. multiple drug regimens), in a therapeutically effective amount. Nox4 inhibitors or the pharmaceutical formulations thereof that are administered simultaneously with said co-agents can be administered in the same or different composition(s) and by the same or different route(s) of administration.

### Patients

In one embodiment, patients according to the invention are patients suffering from osteoporosis.

In a particular embodiment, patients according to the invention are patients suffering from osteoclastogenesis dysfunction.

In another particular embodiment, patients according to the invention are patients suffering from bone resorption of secondary cause selected from Paget's disease, bone loss resulting from immobilization, osteolytic bone metastasis and bone tumours.

In another particular embodiment, patients according to the invention are patients at risk of developing osteoporosis.

### Use according to the invention

In another embodiment, the invention provides Nox4 inhibitors as well as tautomers, geometrical isomers, optically active forms and pharmaceutically acceptable salts thereof for the preparation of a pharmaceutical composition for the treatment or prophylaxis of osteoporosis.

In a particular embodiment, the invention provides Nox4 inhibitors for the preparation of a pharmaceutical composition for the treatment or prophylaxis of osteoclastogenesis dysfunction.

In another embodiment, the invention provides Nox4 inhibitors for the treatment or prophylaxis of osteoporosis.

In another embodiment, the invention provides Nox4 inhibitors for the treatment or prevention of osteoclastogenesis dysfunction.

In another embodiment, the invention provides a method for treating a patient suffering from osteoporosis, the method comprises administering an effective amount of a Nox4 inhibitor or a pharmaceutical formulation thereof in a patient in need thereof.

In another embodiment, the invention provides a method for treating a patient suffering from osteoclastogenesis dysfunction, the method comprises administering an effective amount of a Nox4 inhibitor or a pharmaceutical formulation thereof in a patient in need thereof.

In another embodiment, the invention provides a method for inhibiting osteoclastogenesis in a patient in need thereof, wherein the method comprises administering en affective amount of a Nox4 inhibitor in a patient in need thereof.

In another embodiment, invention provides a method of identifying an inhibitor of ostoeclastogenesis comprising the following steps:
(i) Contacting a substance to be screened with an encoding or expressing system for Nox4;
(ii) Assessing Nox4 activity or Nox-4 expression ability of the said system;
(iii) Comparing the Nox4 activity or Nox4 expression ability in step (ii) with the Nox4 activity or Nox4expression ability in the absence of the substance;
(iv) Selecting a substance for which a decrease in Nox4 activity or Nox4 expression ability in step (iii) is observed.

In a particular embodiment, is provided a method of identifying an inhibitor of ostoeclastogenesis for use in the prevention and/or treatment of an osteoclastogenesis dysfunction and/or osteoporosis.

In another particular embodiment, the method of identifying an inhibitor of ostoeclastogenesis according to the invention is such that the Nox4 activity is assessed in an enzymatic assay, an immunoassay, a Western blotting assay, a ROS formation assay.

In another particular embodiment, the method of identifying an inhibitor of ostoeclastogenesis according to the invention is such that the Nox4 expression ability is assessed by assaying the expression or activity of a gene regulated by Nox4 such as NFAT1c or AP-1.

In another particular embodiment, the method of identifying an inhibitor of ostoeclastogenesis according to the invention is such that the Nox4 expression ability is assessed by Nothern blotting, microarray analysis or RT-PCR.

In another embodiment, the invention provides a pharmaceutical composition containing at least one Nox4 inhibitor according to the invention and a pharmaceutically acceptable carrier, diluent or excipient thereof.

In another embodiment, the invention provides a pharmaceutical composition combining at least one Nox4 inhibitor with at least one co-agent useful in the treatment of osteoporosis and a pharmaceutically acceptable carrier, diluent or excipient thereof.

In a particular embodiment, the compounds, pharmaceutical formulation and methods of the invention are contemplated for use in the prevention and/or treatment of an osteoclastogenesis dysfunction and/or osteoporosis of a patient.

In a particular embodiment, the invention provides Nox4 inhibitors or a use thereof and methods according to the invention wherein the Nox4 inhibitor is to be administered in combination with a co-agent useful in the treatment of osteoporosis or osteoclastogenesis dysfunction.

The compounds of invention have been named according the IUPAC standards used in the program ACD/Name (product version 10.01).

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description and accompanying drawings. Such modifications are intended to fall within the scope of the appended claims.

The invention having been described, the following examples are presented by way of illustration, and not limitation.

### EXAMPLES

The efficacy of Nox4 inhibition in the treatment of osteoporosis is supported by the following experiments.

### The following abbreviations refer respectively to the definitions below:

**eq.** (equivalent), **nm** (nanometer), **BMNC** (bone marrow mononuclear cells), **BSA** (Bovine serum albumin), **DCF** (2,7-dichlorodihydrofluorescein), **DCM** (dichloromethane), **DMEM** (Dulbecco's Modified Eagle Medium), **DMSO** (Dimethyl Sulfoxide), **DAPI** (4,6 Diamidino-2-phenylindole), **DPI** (Diphenyl-iodonium), **EA** (Ethyl Acetate), **EDTA** (ethylenediamine tetraacetic acid), **EGF** (Epidermal Growth Factor), **EGTA** (ethylene glycol tetraacetic acid), **FCS** (Foetal Calf Serum), **GADPH** (Glyceraldehyde 3-phosphate dehydrogenase), **HBSS** (Hank's Buffered Salt Solution), **HEPES** (4-(2-hydroxyethyl)-1-piperazineethanesulfonic acid), **H₂DCF-DA** (2',7'-dichlorodihydro fluorescein diacetate), **M-CSF** (macrophage colony stimulating factor), **MEM** (minimal essential medium), **NADPH** (Nicotinamide adenine dinucelotide diphosphate reduced form), **NBT** (Nitroblue tetrazolium), **PE** (petroleum ether), **TLC** (Thin Layer Chromatography), **TGF-β** (Tumor Growth Factor beta), **ROS** (Reactive oxygen species), **SOD** (Superoxide dismutase), **SYBR** (Sybr Green™ N',N'-dimethyl-N-[4-[(E)-(3-methyl-1,3-benzothiazol-2-ylidene)methyl]-1-phenylquinolin-1-ium-2-yl]-N-propylpropane-1,3-diamine).

### Example 1: Measurement of levels of reactive oxygen species in different cell cultures

The Nox4 activity of Nox4 inhibitors according to the invention may be tested for their activity in the inhibition or reduction of formation of reactive oxygen species (ROS) from oxygen in cell free assays. In order to fully characterise Nox4 inhibitors, Nox subunit-specific cell-free, membrane-based systems have been developed and specific recombinant proteins have been added to membranes heterologously over-expressing their specific isoform. Nox enzymes produce superoxide (O₂·-) as primary product, however due to the high instability and reactivity of this molecule which dismutates naturally into hydrogen peroxide (H₂O₂), therefore, read-out techniques involving probes that do measure H₂O₂ were used (e.g. using Amplex Red as a probe in order to facilitate consistent and accurate measurements). The activity of the compounds is tested in the following cell free assay using the techniques described here below:

### Materials

NADPH (A1395.0100) was from Axonlab. Amplex red (A22177) was purchased from Invitrogen. Horse Radish Peroxidase (HRP) (10108090001) was purchased from Roche.

### Plasmid Construction

Human Nox1 (NM_007052.4), human NOX2 (NM_000397), and human NOX5 (NM_0245052244) cDNAs were cloned into the pcDNA5/TO (Invitrogen). Human p22 (NM_000101.2) was cloned into pcDNA3.1/Zeo (+) (Invitrogen), and human NOXA1 (AY255769.1) and human NOXO1 (AB097667) were cloned into the bi-cistronic pVITRO1-neo-mcs plasmid (Invivogen).

### Cell Culture

Transfected T-REx™-CHO cells (R718-07) purchased from Invitrogen and T-REx™-293 cell line expressing hNOX4 (hNOX4 T-REx™-293) *(*Serrander et al., 2007, Biochem. J., 406, 105-114*)* were cultured in DMEM and in Ham's F12 medium respectively. Both media were supplemented with 10% FBS, 2 mM glutamine, penicillin (100 U/ml) and streptomycin (100 µg/ml). Selection pressure was maintained by continuous inclusion of blasticidin (5 µg/ml), and/or G418 (0.25 mg/ml), and/or Hygromycin (0.25 mg/ml) and/or zeocin (0.025 mg/ml).

### Stable Transfected Cell Line Generation

Cells were transfected by using the FuGENE 6 method. Cells stably expressing functional human Nox1 (hNOX1 T-REx™-CHO) were obtained by co-transfecting T-REx™-CHO cells with human Nox1, human p22, human NOXA1 and human NOXO1. Cells stably expressing functional human Nox2 (hNOX2 T-REx™-CHO) were obtained by co-transfecting T-REx™-CHO cells with human nox2, human p22 and human cofactors. Cells stably expressing functional human Nox5 (hNOX5 T-REx™-CHO) were obtained by transfecting T-REx™-CHO cells with human Nox5. Co-transfected cells were selected with the appropriate antibiotics for 10 days. For the selection, hygromycin and G418 were used at 2 mg/ml and zeocin at 0.1 mg/ml. For the establishment of each stably transfected cell line, a pool of antibiotic-resistant cells was generated. Cells were cloned by limiting dilution. Several clones were established and investigated for ROS production upon tetracycline-induced hNox expression using a cellular AR assay. The expression of NOXes was done with tetracycline (1 µg/ml) during 24 h prior to the assay.

### Membrane Preparation

Human Nox1, Nox2, Nox4, and Nox5 expression were induced with Tetracycline (1 µg/ml) during 24h prior to the membrane preparation Membranes from transfected cells overexpressing Nox1, Nox2, Nox4 or Nox5 were prepared as previously described in Palicz et al., 2001, J. Biol. Chem., 76, 3090-3097*.* After resuspension in sonication buffer (11% sucrose, 120 mM NaCl, 1 mM EGTA in PBS, pH 7.4 for Nox4- or NOX5-expressing cells) or in relax buffer (10 mM Pipes, 3mM NaCl, 3.5 mM MgCl2, 0.1M KCI, pH 7.4), cells were broken by sonication and centrifuged (200 g, 10 min). The supernatant was layered onto a 17/40% (w/v) discontinuous sucrose gradient and centrifuged (150,000 X g for 60 min). Membrane fractions were collected from the 17/40% interface and were stored at -80 °C. Protein concentration was determined with the Bradford reagent.

### ROS production measurement.

Reactive Oxygen Species (ROS) production by membranes expressing either human Nox1,or human Nox2 or human Nox4 or human Nox5 or by xanthine /xanthine oxidase were measured using the Amplex Red (AR) method following a slightly modified version of the manufacturer's instruction manual (Invitrogen). Briefly, membranes or xanthine were incubated in PBS with Amplex Red, Horse Radish Peroxidase (HRP) and appropriated co-factors. ROS production was induced by addition of NADPH to NOX-expressing membrane or by addition of xanthine to xanthine oxidase. Non-specific signal was measured in the absence of membranes or in the absence of xanthine. Antagonist activity of compounds was measured in the presence of increasing concentrations ranging from 1 nM to 100 µM. After an incubation of 20 min in a plate shaker and incubator (Titramax 1000, VWR) at 37°C with weak agitation (300 rpm), ROS levels were measured for 10 min in a BMG Fluostar microplate reader with excitation and emission wavelengths of 544 nm and 590 nm respectively.

Data were analysed using Prism (GraphPad Software Inc., San Diego, CA). K₁ values were calculated using the Cheng-Prusoff equation and represent the average of at least three individual experiments performed in triplicate (Table 1).

**Table 1**

| Compound n° | Ki hNox4 (nM) | Ki hNox2 (nM) |
|---|---|---|
| (1) | 75 | 813 |
| (2) | 48 | 670 |
| (3) | 57 | 735 |
| (4) | 61 | 813 |
| (13) | 80 | 985 |
| (32) | 114 | 1835 |
| (41) | 147 | 1500 |

### Example 2: Nox4 negative effects on bone density in mice by increasing the bone osteoclast content

In order to determine the role of NADPH oxidases for the bone homeostasis, the trabecular density, as measured by peripheral quantitative computed tomography as described below, of knockout mouse lines were compared to their WT litter mates, Nox4-/- exhibited a 30% higher trabecular density of the distal femur (Fig 1A&B) whereas this effect was not observed in Nox2y/- (Nox2y/+: 345.3 ± 12.5 Nox2y/- 329.0 ± 18.9 mg/m³) or Nox1y/- (Noxly/+:262.2 ± 19.7; Nox1: 268.7 ± 11.0 mg/m³) mice which, for the first time supports that only Nox4 but not the other Nox homologues are involved in osteoporosis. A higher bone density of Nox4-/- mice compared to WT mice was also evident by a greater trabecular width and thickness, whereas trabecular number (4.3 ± 0.4 vs. 4.4 ± 0.3 No/mm²; WT vs. Nox4-/-) and separation (217 ± 23 vs. 207 ± 14 mm; WT vs. Nox4-/-) were similar between WT and Nox4-/- mice. As a functional consequence, the stability of the bone (as measured by the three point binding tests as described below) of Nox4-/- mice was significantly higher than that of WT mice (Figure 1C).

The higher bone density of Nox4-/- compared to WT littermates suggests that Nox4 may promote bone resorption or may inhibit bone formation. As estimated from calcein labeling as described below, the bone formation rate was similar between WT and Nox4-/- mice and also the mineral apposition rate and double labeled surface were not different between WT and Nox4-/- mice. Consistently, plasma levels of procollagen1 N-terminal peptide, a marker of osteoblast activity (as measured by ELISA assay as described below), was similar in the plasma of WT and Nox4-/- mice (Figure 2A). This data exclude a Nox4-dependent inhibition of osteoblasts as the mechanism underlying the increased bone density of Nox4-/- mice and point towards an effect of osteoclasts. Indeed, markers of bone resorption like TRAP 5b and carboxy-terminal collagen crosslinks (CTX) were significantly lower in the plasma of Nox4-/- compared to WT mice (Figure 2B,C) which points towards higher osteoclast activity or abundance in WT animals. Indeed, immunohistological analysis for TRAP positive cells as described below in the bone demonstrated that Nox4-/- mice had an approx. 50% reduction in the number of osteoclasts (N. Oc) compared to WT animals (Figure 1D/E). Accordingly, comparison of the bone proteins revealed a lower expression of the osteoclast marker protein osteoclast-associated receptor (OSCAR) and higher expression of the osteoblast protein osteopontin in the bones of Nox4-/- compared to WT animals (Figure 1F). These observations suggest that genetic deletion of Nox4 results in a reduced formation or increased apoptosis of osteoclasts.

### Mice and animal procedures.

Nox4-null mice were generated by targeted deletion of the translation initiation site and exons 1 and 2 of the Nox4 gene (Genoway) as described in Zhang et al., 2010, Proc. Natl. Acad. Sci. U. S. A 107:18121-18126*.* Briefly, a 5' murine Nox4 genomic DNA fragment was isolated from a 129sv DNA BAC library and used to generate a targeting construct containing exons 1 and 2 flanked by 1oxP sites, a negative-selection diptheria toxin A cassette and a positive selection neomycin cassette flanked by Flippase Recognition Target sites. The targeting construct was electroporated into 129sv embryonic stem cells, recombinant clones identified by PCR and Southern blot analysis, and injected into C57BL/6 blastocysts. Heterozygous mice obtained from germline chimeras were bred with C57BL/6 Cre-deletor mice and Flp-deletor mice to generate heterozygous knockout mice. Nox4-null animals were obtained by intercrossing progeny and were backcrossed >10 generations with C57BL/6 mice.

Bone composition was studied after 6 weeks. Bone formation was determined by the calcein method where animals were subcutaneously injected with calcein (20 mg/kg body weight) which was injected on days 2 and 1 before sacrifice of the mice.

### Biochemistry

Serum concentrations of Procollagen 1 N-terminal peptide PINP (DRG Diagnostics), TRAP-5b (DRG Diagnostics) and CTX (USCNK, Life Science Inc.) were assessed by a mouse-specific enzyme-linked immunosorbent assay (ELISA) according to the manufacturer's instructions.

### Bone mineral density (BMD) measurements

BMD of the left femur was measured by peripheral quantitative computed tomography (QCT) using an XCT Research QCT machine (Stratec Medizintechnik, Pforzheim, Germany). One slice (0.2 mm thick) in the mid-diaphysis of the femur and 3 slices in the distal femoral metaphysis located 1.5, 2, and 2.5 mm proximal to the articular surface of the knee joint were measured. BMD values of the distal femoral metaphysic were calculated as the mean of 3 slices. A voxel size of 0.070 mm and a threshold of 710 mg/cm³ were used for calculation of BMD as described in Hofbauer et al., 2009, Arthritis Rheum., 60(5):1427-1437*.*

### Bone histology and histomorphometry

Processing of bone specimens and cancellous bone histomorphometry in the distal femoral metaphysis was performed on calcified bone sections of the distal femoral metaphysis and quantified using OsteoMeasure 3.0 (Osteometrics, Atlanta, GA) and AxioVision 4.6 (Carl Zeiss, Oberkochen, Germany) software for image analysis as described *in* Reim al., 2008, J. Bone Miner. Res., 23(5):694-704*.*

### Analysis of bone biomechanics

The femurs were loaded to failure by 3-point bending tests using a Zwick Z020/TN2A materials testing machine (Zwick, Ulm, Germany) with a 1 kN force detector, and a force resolution of 0.01N. The distance between the lower supports for the bending tests was 5 mm. Crosshead speed during testing was 0.2 mm/minute for all measurements. From the force-displacement data, ultimate force (Fmax; in N) was calculated as described in Binder et al., 2009, Nature Medicine,15: 417-424*.*

### Example 3: ROS formation increase during osteoclastogenesis in a Nox4-dependent manner

To study the role of Nox4 on osteoclast formation, Nox4 expression was determined in the course of differentiation as well as the effect of genetic knockout of Nox4 on the differentiation process in a murine *ex-vivo* differentiation assay as described below. When human peripheral blood mononuclear cells (PBMC) were differentiated into osteoclasts, a time-dependent increase in the expression of Nox1 and Nox4 was observed over the 21 days protocol (Figure 3).

*In vitro* osteoclastogenesis was performed by stimulating bone marrow mononuclear cells (BMNC) with M-CSF and RANKL as described below and differentiated osteoclasts were identified by TRAP staining as described in Mukherjee et al., 2008, J. Clin. Invest., 118:491-504 using protocol 387A-1KT; Sigma-Aldrich (www.sigma-aldrich.com). This approach resulted in an approximately 50% lower osteoclast formation in the Nox4-/- cells as compared to the WT group (Figure 1G,H). This suggests that the lower number of osteoclasts in the bone sections is due to an impaired differentiation. In order to provide some functional evidence for a role of Nox4, ROS formation was measured. As already suggested by the increase in Nox4 expression during differentiation of human PBMCs, ROS formation after stimulation of primed BMNC with RANKL increased (Figure 1I).

Importantly, this effect was less much pronounced and did not reach statistical significance in cells derived from Nox4-/- mice, clearly demonstrating that Nox4 is mediating the increase in ROS formation in response to RANKL in the course of differentiation.

### Cell culture

Human osteoclasts were generated as described in Rauner et al., 2011, Endocrinology 2011;152(1):103-112*.* Briefly, peripheral blood mononuclear cells (PBMCs) were collected from buffy coats provided by the local blood bank using Ficoll (1.077 g/ml, 95 Biochrom) centrifugation. Following attachment in α-MEM (Invitrogen) containing 10% FCS (PAA) and 1% penicillin/streptomycin, medium was replaced with basal medium supplemented with 25 ng/ml M-CSF (R&D Systems) for 3 days. Thereafter, cells were cultured for up to 21 days in differentiation medium containing 25 ng/ml M-CSF and 50 ng/ml RANKL (R&D Systems) for osteoclastogenesis. To induce osteoblastic differentiation, cells were cultured in growth medium supplemented with L-ascorbate phosphate (100 µM), β-glycerol phosphate (10 mM), and dexamethasone (10 nM).

### Murine osteoclast differentiation ex vivo

Epiphyseal ends of the femora and tibiae were cut off and the bone marrow was flushed out with DMEM containing 10% FCS. Bone marrow stromal cells were collected and plated at a density of 2 x 106 cells/cm² in α-MEM containing 10% FCS and 1% penicillin/streptomycin. Following attachment, medium was replaced by basal medium supplemented with 25 ng/ml M-CSF (R&D Systems) for 2 days. Thereafter, cells were cultured for additional 6 days in differentiation medium containing 25 ng/ml M-CSF and 50 ng/ml RANKL (R&D Systems). TRAP staining was performed using the leukocyte acid phosphatase kit (Sigma) following the manufactures instructions according to *Mukherjee et al., 2008, supra.*

### Amplex Red assay for H₂O₂ production

ROS production was estimated according to Schröder et al., 2006, Circ. Res., 105(6):537-544 with minor modifications. Cells were grown on 12 well plates and differentiated with or without RANKL as described above. Following a washing step cells were incubated with HEPES Tyrode containing Amplex Red (50 µmol/L, Invitrogen), horse-radish peroxidase (2 U/mL) and no BSA. After 45 min the supernatant was transferred to 96 well plates and H₂O₂-dependent oxidation of Amplex Red was measured in a microplate fluorimeter (excitation 540 nm, emission 580 nm). Experiments were performed in the presence and absence of catalase (50 U/mL). H₂O₂ formation was determined as the catalase-sensitive part of the Amplex Red oxidation.

### Quantitative PCR

RNA was isolated using the HighPure RNA extraction kit from Roche according to the manufacturer's protocol. Five-hundred ng RNA were reverse transcribed using Superscript II (Invitrogen) and subsequently used for SYBR™ green-based real-time PCR reactions using a standard protocol (Applied Biosystems). The primer sequences were as follows (5'-3'):
***mouse β-actin-forward:*** gatctggcaccacaccttct, reverse: ggggtgttgaaggtctcaaa;
***nox4-forward:*** tctcaggtgtgcatgtagcc, reverse: ttctgggatcctcattctgg;
***Nox1-forward:*** 5' aaagccattggatcacaacc 3'
***Nox1-reverse:*** 5' cagaagcgagagatccatcc 3'.

PCR conditions were 95°C for 10 min followed by 30 cycles with 95°C for 10 s and 56°C for 60 s. Expression levels were normalized to β-actin.

### Example 4: Role of Nox4 on RANK signalling

In order to determine the mechanisms underlying the reduced osteoclastogenesis in Nox4-/- mice, the role of this NADPH oxidase in RANK signaling was investigated. The expression of RANK receptor was not different between M-CSF primed BMNC from wild-type and Nox4 -/- mice pointing towards Nox4-mediated differences in RANKL signaling between wild-type and Nox4 -/- mice.

The role of Nox4 for the RANKL-induced increase in cytosolic Ca²⁺ was investigated by FURA2 fluorescence as described below. An incubation of murine M-CSF-primed BMNC with RANKL (30 hours) induced a marked increase in cytosolic Ca²⁺ in cells from WT mice. In contrast, this effect was not observed in cells obtained from Nox4 deficient animals (Figure 4A).

Pharmacological inhibition of Nox4 was achieved by a NOX4 inhibitor, 2-(2-chlorophenyl)-4-[3-(dimethylamino)phenyl]-5-methyl-1H-pyrazolo[4,3-c]pyridine-3,6 (2H,5H)-dione presenting an IC₅₀ approximately 10 fold higher for Nox4 than for Nox2 and no antioxidant activity as measured in an assay of Example 1 (60 mg/kg/day) which was administered by daily gavage for 6 weeks whereas genetic deletion of Nox4 was induced by injection of tamoxifen (40 mg/kg intraperitoneally for 3 consecutive days, before ovariectomy as described above). The control group received daily gavages by the solvent.

Pharmacological inhibition of Nox4 by the NOX4 inhibitor (20 µmol/L), when co-applied with RANKL during the stimulation prevented the RANKL induced increase in cytosolic Ca²⁺ (Figure 4B), similarly as genetic deletion of Nox4, clearly demonstrating that lack of Nox4 activity but not some undefined genetic peculiarities of Nox4-/- mice account for the deficient of RANKL signaling in Nox4-/- cells.

Osteoclast differentiation in response to calcium is mediated by the transcription factor NFAT1c which is translocated into the nucleus after activation by the calcium-sensitive phosphatase calcineurin or partial cleavage by the calcium-activated protease β-Calpain. Western blot analysis as described below demonstrated that the abundance of active µ-Calpain increased in response to RANKL in M-CSF primed murine BMNC which was paralleled by a nuclear accumulation of NFAT1c. Importantly, this effect was absent in cells obtained for Nox4-/- mice, whereas calcineurin level were similar between cells obtained from WT and Nox4-/- mice. These data demonstrate that Nox4, via its action of intracellular calcium is a central mediator of RANK signaling and required for NFAT1c-induced gene expression.

### Ca²⁺ measurement

Cells were grown on 96 well plates and differentiated with or without RANKL as described above. Following a washing step cells were loaded with 1.5 µM Fura-2 (Invitrogen) for 60 min. After another washing step intracellular free Fura-2 and Ca²⁺-bound Fura-2 was measured at the wavelength 340 and 380 nm by the ELISA Reader EnVision (PerkinElmer) in 100 µl HEPES Tyrode. Measurements were repeated 3 times after step by step addition of 40 µL 20 µM Ionomycin, 100 mM EGTA and saturated MnCl₂. Eventually the intracellular Ca²⁺ was calculated as described by Grynkiewicz et al., 1985, J. Biol. Chem., 260(6):3440-3450*.*

### Immunoblotting

Western blot analyses were performed with an infrared-based detection system (Odyssey, Licor, Bad Homburg, Germany). All primary antibodies were purchased from Cell signaling and infrared-fluorescent-dye-conjugated secondary antibodies were obtained from Licor (Bad Homburg). The following lysis buffer was used (pH 7.4, concentrations in mmol/l): Tris-HCl (50), NaCl (150), sodium pyrophosphate (10), sodium fluoride (20), nonidet P40 (1%), sodium desoxycholate (0.5%), proteinase inhibitor mix, phenylmethylsulfonyl fluoride (1), orthovanadate (2), okadaic acid (0.00001).

### Example 5: Nox4 increase in human osteoporosis and NOX4 inhibition effect on prevention of osteoporosis in mice

Osteoporosis is the consequence of an imbalance between osteoblast and osteoclast activity. As the above data supports the role of Nox4 in osteoclastic activity, the possible role of this NADPH oxidase in human osteoporosis was investigated. Human bone specimens from healthy patients and osteoporotic patients or patients with Paget's disease (the two latter disorders being known to be associated with high osteoclast activity) were stained for Nox4 and subsequently subjected to image analysis as described below.

The staining intensity for Nox4 was significantly higher in osteoporotic bone as compared to healthy bone and in Paget's patients than healthy group (Figure 5A). These data suggest that Nox4 also contributes to bone loss in humans and is a mediator of osteoporosis.

To study the latter effect, female mice were subjected to ovariectomy to induce osteoporosis and the effect of pharmacological inhibition of Nox4 with a Nox4 inhibitor as well as acute genetic knockout of the protein was studied. For this, mice were subjected to ovariectomy 8 weeks after birth and treatment was started 2 days after the operation and continued throughout the study.

As expected, 6 weeks after ovariectomy bone density was reduced in control mice. Mice that received pharmacological inhibition exhibited a significantly greater bone density than those that received solvent treatment (Figure 5C).

In order to ascertain that changes in Nox4 activity can be therapeutically useful in osteoporosis, Nox4 was acutely knocked out in Nox4-Flox-Flox mice crossed with ERT2-Cre-tamoxifen deleter mice as described below as follows: animals were subj ected to ovariectomy, treated with 3 inj ections of tamoxifen (40 mg/kg dissolved in sterile corn oil, i.p.) two days before the operation and bones were analyzed 6 weeks later. As reported previously, as a consequence of the partial agonist activity of tamoxifen on the estrogen receptor, the tamoxifen itself attenuated the loss of trabecular density. Despite this, the additional knockout of Nox4 had an additive beneficial effect (Figure 5C).

Overall the above results provide support for an unexpected direct link between Nox4 and bone loss by showing that Nox4 expression is upregulated in human osteoporotic bone sections and that *in vivo* inhibition of Nox4 (pharmacologically or genetically) prevents bone loss in the model of ovariectomy in mice. Therefore, it supports that the loss of bone mass in osteoporosis could be prevented or at least reduced by inhibiting Nox4.

### Bone Immunostaining

Anonymized human bone biopsis histological sections obtained for diagnostic purpose were stained by immunohistochemistry using an anti-Nox4 antibody *(*Szöcs et al., 2002, Arterioscler. Thromb. Vasc. Biol., 22:21-7*).* For this, paraffin sections of de-calficied bone were sectioned to 5µmeter, deparaffinized, rehydrated and after antigen demasking by cooking in citrate buffer (Sigma-Adrich) incubate with the primary antibody (1:300 diluted, dissolved in phosphate-buffered saline containing 0.5% Tween) for 60 min at room temperature. Subsequent, the antibody was washed of and samples and staining was developed with the ABC-vector stain HRP-kit (VECTOR) and counterstained with hemalaun.

### Mice and animal procedures.

Nox4-null mice were generated by targeted deletion of the translation initiation site and exons 1 and 2 of the Nox4 gene (Genoway) as described in Zhang et al., 2010, Proc. Natl. Acad. Sci. U. S. A 107:18121-18126*.* Briefly, a 5' murine Nox4 genomic DNA fragment was isolated from a 129sv DNA BAC library and used to generate a targeting construct containing exons 1 and 2 flanked by loxP sites, a negative-selection diptheria toxin A cassette and a positive selection neomycin cassette flanked by Flippase Recognition Target sites. The targeting construct was electroporated into 129sv embryonic stem cells, recombinant clones identified by PCR and Southern blot analysis, and injected into C57BL/6 blastocysts. Heterozygous mice obtained from germline chimeras were bred with C57BL/6 Cre-deletor mice and Flp-deletor mice to generate heterozygous knockout mice. Nox4-null animals were obtained by intercrossing progeny and were backcrossed >10 generations with C57BL/6 mice.

### Example 6: NOX4 inhibitors

The following compounds listed in Table 2 below are examples of Nox4 inhibitor that could be used according to the invention:

**Table 2**

| **Compound** | **Structure** | **Name** |
|---|---|---|
| **1** | 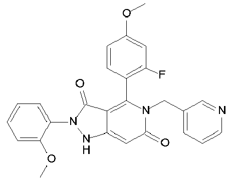 | 4-(2-fluoro-4-methoxyphenyl)-2-(2-methoxyphenyl)-5-(pyridin-3-ylmethyl)-1 H-pyrazolo[4,3-c]pyridine-3,6(2H,5H)-dione |
| **2** | 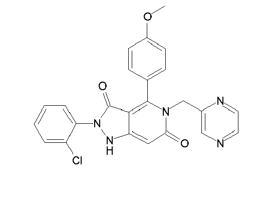 | 2-(2-chlorophenyl)-4-(4-methoxyphenyl)-5-(pyrazin-2-ylmethyl)-1 H-pyrazolo[4,3-c]pyridine-3,6(2H,5H)-dione |
| **3** | 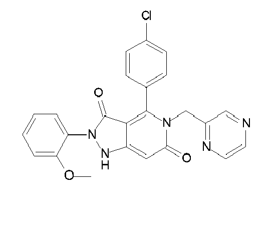 | 4-(4-chlorophenyl)-2-(2-methoxyphenyl)-5-(pyrazin-2-ylmethyl)-1 H-pyrazolo[4,3-c]pyridine-3,6(2H,5H)-dione |
| **4** | 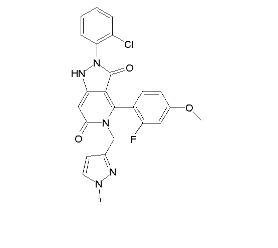 | 2-(2-chlorophenyl)-4-(2-fluoro-4-methoxyphenyl)-5-[(1-methyl-1H-pyrazol-3-yl)methyl]-1 H-pyrazolo[4,3-c]pyridine-3,6(2H,5H)-dione |
| **5** | 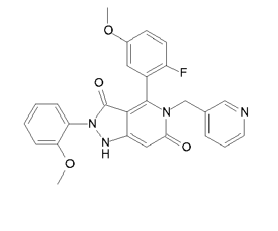 | 4-(2-fluoro-5-methoxyphenyl)-2-(2-methoxyphenyl)-5-(pyridin-3-ylmethyl)-1 H-pyrazolo[4,3-c]pyridine-3,6(2H,5H)-dione |
| **6** | 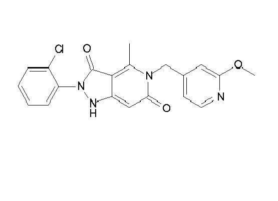 | 2-(2-chlorophenyl)-5-[(2-methoxypyridin-4-yl)methyl]-4-methyl-1H-pyrazolo[4,3-c]pyridine-3,6(2H,5H)-dione |
| **7** | 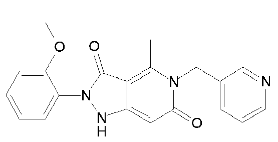 | 2-(2-methoxyphenyl)-4-methyl-5-(pyridin-3-ylmethyl)-1H-pyrazolo[4,3-c]pyridine-3,6(2H,5H)-dione |
| **8** | 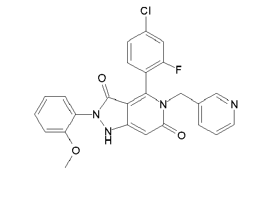 | 4-(4-chloro-2-fluorophenyl)-2-(2-methoxyphenyl)-5-(pyridin-3-ylmethyl)-1 H-pyrazolo[4,3-c]pyridine-3,6(2H,5H)-dione |
| **9** | 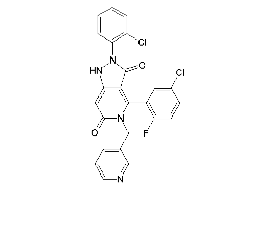 | 4-(5-chloro-2-fluorophenyl)-2-(2-chlorophenyl)-5-(pyridin-3-ylmethyl)-1H-pyrazolo[4,3-c]pyridine-3,6(2H,5H)-dione |
| **10** | 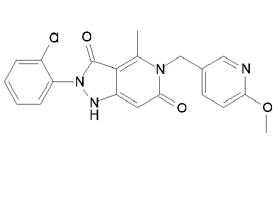 | 2-(2-chlorophenyl)-5-[(6-methoxypyridin-3-yl)methyl]-4-methyl-1H-pyrazolo[4,3-c]pyridine-3,6(2H,5H)-dione |
| **11** | 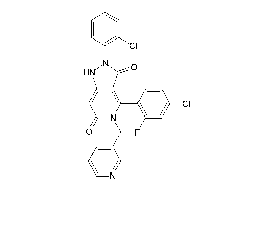 | 4-(4-chloro-2-fluorophenyl)-2-(2-chlorophenyl)-5-(pyridin-3-ylmethyl)-1H-pyrazolo[4,3-c]pyridine-3,6(2H,5H)-dione |
| **12** | 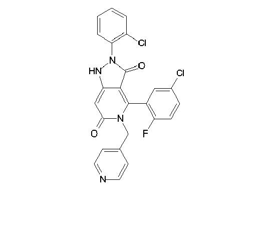 | 4-(5-chloro-2-fluorophenyl)-2-(2-chlorophenyl)-5-(pyridin-4-ylmethyl)-1H-pyrazolo[4,3-c]pyridine-3,6(2H,5H)-dione |
| **13** | 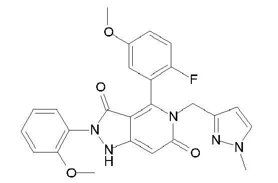 | 4-(2-fluoro-5-methoxyphenyl)-2-(2-methoxyphenyl)-5-[(1-methyl-1H-pyrazol-3-yl)methyl]-1H-pyrazolo[4,3-c]pyridine-3,6(2H,5H)-dione |
| **14** | 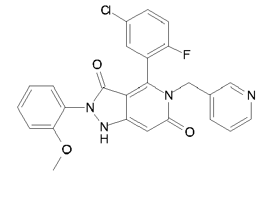 | 4-(5-chloro-2-fluorophenyl)-2-(2-methoxyphenyl)-5-(pyridin-3-ylmethyl)-1H-pyrazolo[4,3-c]pyridine-3,6(2H,5H)-dione |
| **15** | 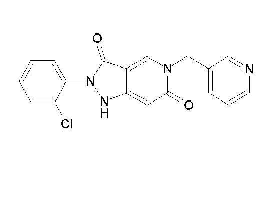 | 2-(2-chlorophenyl)-4-methyl-5-(pyridin-3-ylmethyl)-1H-pyrazolo[4,3-c]pyridine-3,6(2H,5H)-dione |
| **16** | 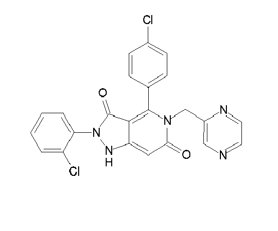 | 2-(2-chlorophenyl)-4-(4-chlorophenyl)-5-(pyrazin-2-ylmethyl)-1H-pyrazolo[4,3-c]pyridine-3,6(2H,5H)-dione |
| **17** | 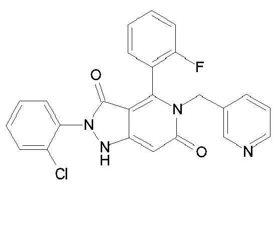 | 2-(2-chlorophenyl)-4-(2-fluorophenyl)-5-(pyridin-3-ylmethyl)-1 H-pyrazolo[4,3-c]pyridine-3,6(2H,5H)-dione |
| **18** | 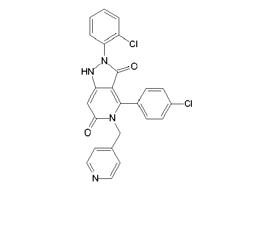 | 2-(2-chlorophenyl)-4-(4-chlorophenyl)-5-(pyridin-4-ylmethyl)-1H-pyrazolo[4,3-c]pyridine-3,6(2H,5H)-dione |
| **19** | 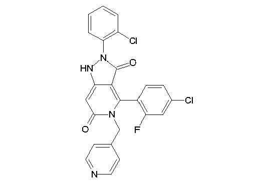 | 4-(4-chloro-2-fluorophenyl)-2-(2-chlorophenyl)-5-(pyridin-4-ylmethyl)-1H-pyrazolo[4,3-c]pyridine-3,6(2H,5H)-dione |
| **20** | 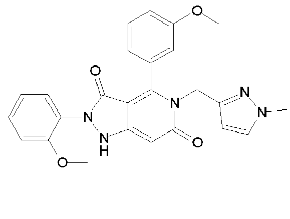 | 2-(2-methoxyphenyl)-4-(3-methoxyphenyl)-5-[(1-methyl-1H-pyrazol-3-yl)methyl]-1H-pyrazolo[4,3-c]pyridine-3,6(2H,5H)-dione |
| **21** | 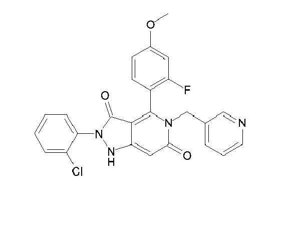 | 2-(2-chlorophenyl)-4-(2-fluoro-4-methoxyphenyl)-5-(pyridin-3-ylmethyl)-1H-pyrazolo[4,3-c]pyridine-3,6(2H,5H)-dione |
| **22** | 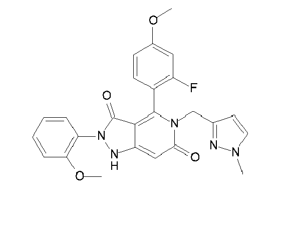 | 4-(2-fluoro-4-methoxyphenyl)-2-(2-methoxyphenyl)-5-[(1-methyl-1H-pyrazol-3-yl)methyl]-1H-pyrazolo[4,3-c]pyridine-3,6(2H,5H)-dione |
| **23** | 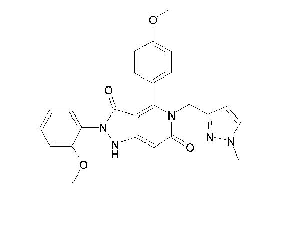 | 2-(2-methoxyphenyl)-4-(4-methoxyphenyl)-5-[(1-methyl-1H-pyrazol-3-yl)methyl]-1H-pyrazolo[4,3-c]pyridine-3,6(2H,5H)-dione |
| **24** | 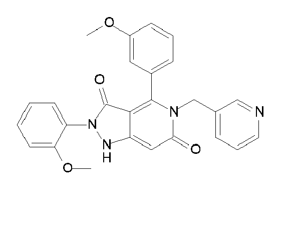 | 2-(2-methoxyphenyl)-4-(3-methoxyphenyl)-5-(pyridin-3-ylmethyl)-1H-pyrazolo[4,3-c]pyridine-3,6(2H,5H)-dione |
| **25** | 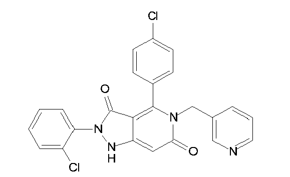 | 2-(2-chlorophenyl)-4-(4-chlorophenyl)-5-(pyridin-3-ylmethyl)-1H-pyrazolo[4,3-c]pyridine-3,6(2H,5H)-dione |
| **26** | 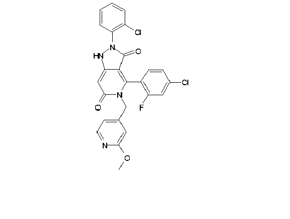 | 4-(4-chloro-2-fluorophenyl)-2-(2-chlorophenyl)-5-[(2-methoxypyridin-4-yl)methyl]-1H-pyrazolo[4,3-c]pyridine-3,6(2H,5H)-dione |
| **27** | 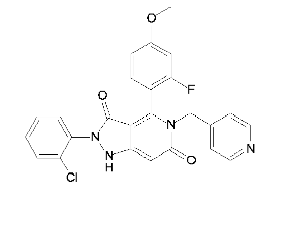 | 2-(2-chlorophenyl)-4-(2-fluoro-4-methoxyphenyl)-5-(pyridin-4-ylmethyl)-1H-pyrazolo[4,3-c]pyridine-3,6(2H,5H)-dione |
| **28** | 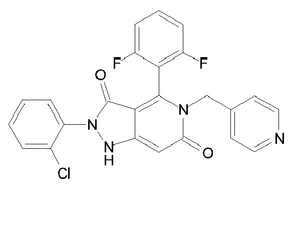 | 2-(2-chlorophenyl)-4-(2,6-difluorophenyl)-5-(pyridin-4-ylmethyl)-1H-pyrazolo[4,3-c]pyridine-3,6(2H,5H)-dione |
| **29** | 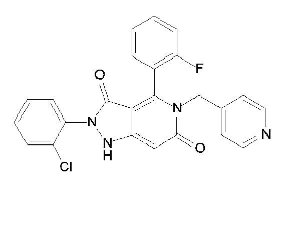 | 2-(2-chlorophenyl)-4-(2-fluorophenyl)-5-(pyridin-4-ylmethyl)-1H-pyrazolo[4,3-c]pyridine-3,6(2H,5H)-dione |
| **30** | 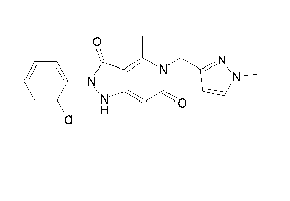 | 2-(2-chlorophenyl)-4-methyl-5-[(1-methyl-1 H-pyrazol-3-yl)methyl]-1H-pyrazolo[4,3-c]pyridine-3,6(2H,5H)-dione |
| **31** | 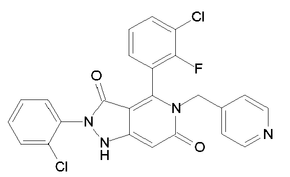 | 4-(3-chloro-2-fluorophenyl)-2-(2-chlorophenyl)-5-(pyridin-4-ylmethyl)-1H-pyrazolo[4,3-c]pyridine-3,6(2H,5H)-dione |
| **32** | 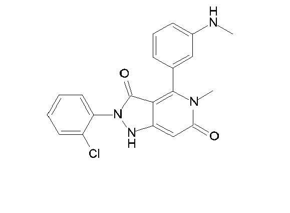 | 2-(2-chlorophenyl)-5-methyl-4-[3-(methylamino)phenyl]-1H-pyrazolo[4,3-c]pyridine-3,6(2H,5H)-dione |
| **33** | 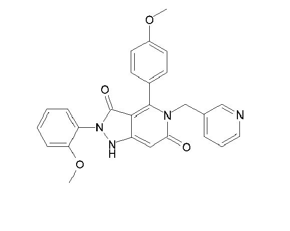 | 2-(2-methoxyphenyl)-4-(4-methoxyphenyl)-5-(pyridin-3-ylmethyl)-1H-pyrazolo[4,3-c]pyridine-3,6(2H,5H)-dione |
| **34** | 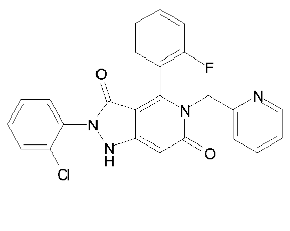 | 2-(2-chlorophenyl)-4-(2-fluorophenyl)-5-(pyridin-2-ylmethyl)-1H-pyrazolo[4,3-c]pyridine-3,6(2H,5H)-dione |
| **35** | 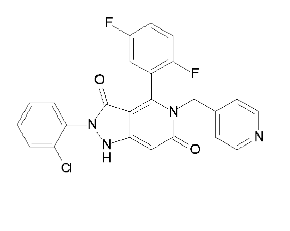 | 2-(2-chlorophenyl)-4-(2,5-difluorophenyl)-5-(pyridin-4-ylmethyl)-1H-pyrazolo[4,3-c]pyridine-3,6(2H,5H)-dione |
| **36** | 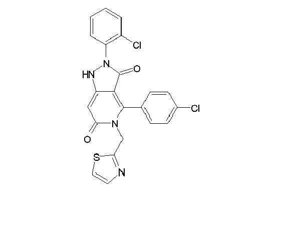 | 2-(2-chlorophenyl)-4-(4-chlorophenyl)-5-(1,3-thiazol-2-ylmethyl)-1H-pyrazolo[4,3-c]pyridine-3,6(2H,5H)-dione |
| **37** | 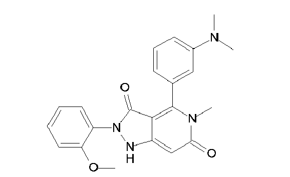 | 4-[3-(dimethylamino)phenyl]-2-(2-methoxyphenyl)-5-methyl-1H-pyrazolo[4,3-c]pyridine-3,6(2H,5H)-dione |
| **38** | 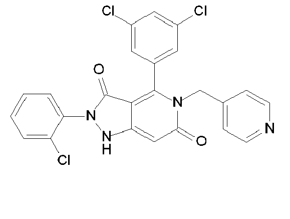 | 2-(2-chlorophenyl)-4-(3,5-dichlorophenyl)-5-(pyridin-4-ylmethyl)-1H-pyrazolo[4,3-c]pyridine-3,6(2H,5H)-dione |
| **39** | 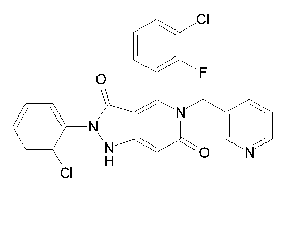 | 4-(3-chloro-2-fluorophenyl)-2-(2-chlorophenyl)-5-(pyridin-3-ylmethyl)-1H-pyrazolo[4,3-c]pyridine-3,6(2H,5H)-dione |
| **40** | 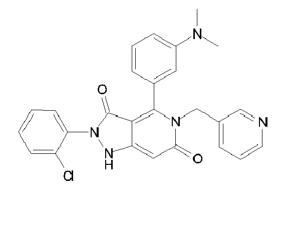 | 2-(2-chlorophenyl)-4-[3-(dimethylamino)phenyl]-5-(pyridin-3-ylmethyl)-1H-pyrazolo[4,3-c]pyridine-3,6(2H,5H)-dione |
| **41** | 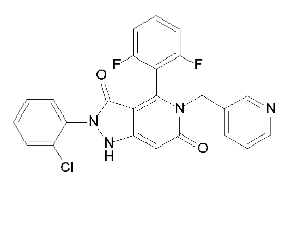 | 2-(2-chlorophenyl)-4-(2,6-difluorophenyl)-5-(pyridin-3-ylmethyl)-1H-pyrazolo[4,3-c]pyridine-3,6(2H,5H)-dione |
| **42** | 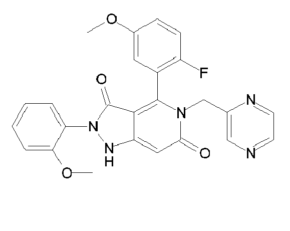 | 4-(2-fluoro-5-methoxyphenyl)-2-(2-methoxyphenyl)-5-(pyrazin-2-ylmethyl)-1H-pyrazolo[4,3-c]pyridine-3,6(2H,5H)-dione |
| **43** | 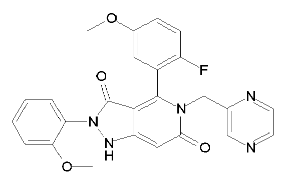 | 2-(2-chlorophenyl)-4-(2,5-difluorophenyl)-5-(pyridin-3-ylmethyl)-1H-pyrazolo[4,3-c]pyridine-3,6(2H,5H)-dione |

### Synthesis of NOX4 inhibitors

The NOX4 inhibitors above can be prepared from readily available starting materials using the following method for compound (1) detailed below. It will be appreciated that where typical or preferred experimental conditions (i.e. reaction temperatures, time, moles of reagents, solvents etc.) are given, other experimental conditions can also be used unless otherwise stated. Optimum reaction conditions may vary with the particular reactants or solvents used, but such conditions can be determined by the person skilled in the art, using routine optimisation procedures. If synthetic method below is not applicable and/or necessary intermediates for the synthesis of compounds, suitable methods of preparation known by a person skilled in the art should be used. In general, the synthesis pathways for any individual compound will depend on the specific substituents of each molecule and upon the ready availability of intermediates necessary; again such factors being appreciated by those of ordinary skill in the art. For all the protection and deprotection methods, see Philip J. Kocienski, in "Protecting Groups ", Georg Thieme Verlag Stuttgart, 2005 and Theodora W. Greene and Peter G. M. Wuts in "Protective Groups in Organic Synthesis ", Wiley Interscience, 4th Edition 2006*.*

Compounds of this invention can be isolated in association with solvent molecules by crystallization from evaporation of an appropriate solvent.

### Formation of 4-(2-fluoro-4-methoxy phenyl)-2-(2-methoxy phenyl)-5-(pyridin-3-yl methyl)-1H-pyrazolo [4,3-c]pyridine-3,6 (2H,5H)-dione (Compound 1)

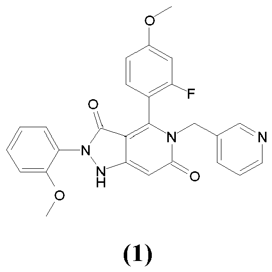

### a) 2-fluoro-4-methoxybenzoyl chloride (Compound B)

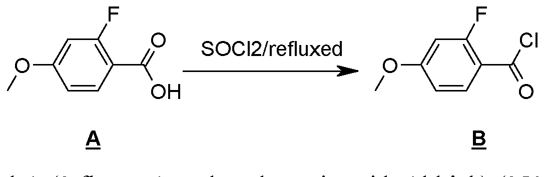

Starting compound A (2-fluoro-4-methoxybenzoic acid, Aldrich) (350 g, 2.06 vmol) in SOCl₂ (2500 ml) was refluxed under N₂ overnight. The reaction was monitored by TLC (72h). The desired intermediate **B** was distilled from the reaction mixture (140°C, 10 mmHg) as white solid (365 g, yield: 94%).

### b) methyl [5-hydroxy-1-(2-methoxyphenyl)-1H-pyrazol-3-yl]acetate (Compound D)

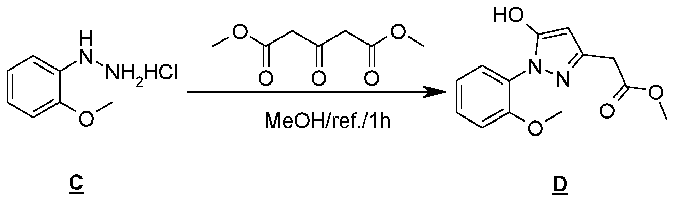

To a solution of starting compound C (2-methoxy phenyl hydrazine hydrochloride, Aldrich) (350 g, 1 eq.) in MeOH (2 L) was added 3-oxo-pentanedioic acid dimethyl ester (Aldrich) (1.2 eq.) under N₂, this solution was been refluxing for 1h, TLC showed there was no STM. The solvent was removed by evaporation and the residue was purified by silica column (DCM/MeOH 200:1∼50:1) to give desired intermediate D as white solid. (360 g, yield 68%).

### c) methyl {4-[(2-fluoro-4-methoxyphenyl)carbonyl]-5-hydroxy-1-(2-methoxyphenyl)-1H-pyrazol-3-yl}acetate (Compound E)

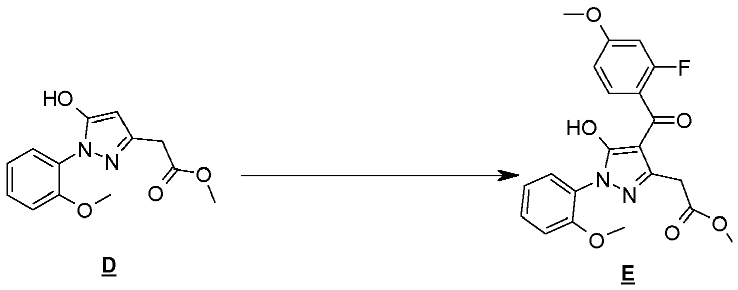

Intermediate **D** (20 g, 1 eq.) obtained above was suspended in dioxane (1200 ml) and then Ca(OH)₂ (15 eq.) was added under N₂. The reaction mixture was heated up to 110°C, then 2-fluoro-4-methoxybenzoyl chloride (compound B, 0.9 eq.) obtained above was dissolved in dioxane (100 mL) and added to the mixture dropwise. The reaction was stirred at 110°C and monitored by LCMS until disappearance of starting material (t=4h). The mixture was filtered and the residue was washed with ethyl acetate (5*150 mL). The combined filtrate was concentrated and recrystallized with PE to yield crude intermediate product **E** as yellow power without further purification (11.3 g, yield: 36.9%).

### d) methyl [(4E)-4-{(2-fluoro-4-methoxyphenyl)[(pyridin-3-ylmethyl)amino] methylidene}-1-(2-methoxyphenyl)-5-oxo-4, 5-dihydro-1H-pyrazol-3-yl]acetate (Compound F)

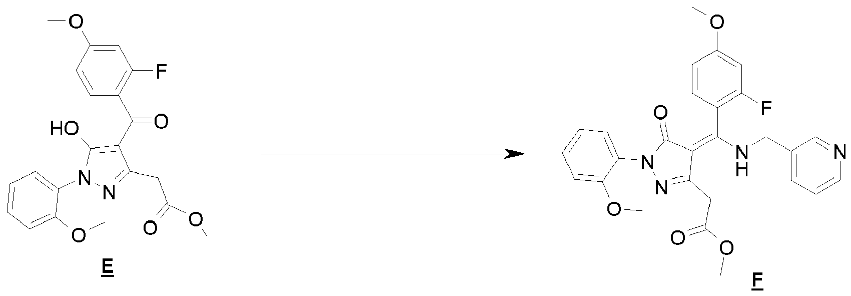

Intermediate E obtained above (20 g, 1 eq.) was dissolved in toluene (500 mL), TiCl₄ (2 mL) and AcOH (100 ml) were added at 90°C under N₂. After addition of 1-pyridin-3-yl methanamine (2.0 eq.) (Aldrich), the reaction mixture was stirred at 100°C and monitored by LCMS showing the disappearance of the starting material (1.5 h). The reaction mixture was evaporated until dryness. The residue was dissolved in EA (500 ml), washed with saturated NaHCO₃ solution, dried over Na₂SO₄ and concentrated. A white solid of compound **F** was yielded by recrystallization from DCM and PE (9.5 g, yield: 37.9%).

### e) 4-(2-fluoro-4 methoxy phenyl)-2-(2-methoxy phenyl)-5-(pyridin-3-ylmethyl)-1H pyrazolo [4,3-c]pyridine-3,6 (2H,5H)-dione (Compound 1)

The enamine intermediate **F** obtained above (160 g, 1 eq.) was treated with freshly prepared MeONa in MeOH (2M, 1000 ml), the solution was stirred at room temperature until disappearance of the starting enamine (t=1h). The reaction mixture was concentrated to eliminate MeOH and the crude was dissolved in water (800 ml), extracted with DCM (300 ml*6). Then the water phase was acidified with HCl aq to pH=5∼6, then extracted with DCM (300 ml*3). The combined organic layer was dried over Na₂SO₄, concentrated *in vacuo* to give final product compound (1) as yellow solid (110 g, yield: 68%).

## Claims

1. A Nox4 inhibitor for the prevention and/or treatment of an osteoclastogenesis dysfunction and/or osteoporosis.

2. A Nox4 inhibitor according to claim 1 wherein Nox4 inhibitor presents an inhibitory constant (Ki) for Nox4 inhibitory activity ranging from about 10 nM or lower to 500 nM in functional assay of ROS production.

3. A Nox4 inhibitor according to claims 1 or 2 for the prevention and/or treatment of osteoporosis.

4. A Nox4 inhibitor according to claims 1 or 2 for the prevention and/or treatment of an osteoporotic or a pre-osteoporotic osteoclastogenesis dysfunction.

5. A Nox4 inhibitor according to claims 1 or 2 for the prevention and/or treatment of bone resorption of secondary cause selected from Paget's disease, bone loss resulting from immobilization, osteolytic bone metastasis and bone tumours.

6. A Nox4 inhibitor according selected from the following group:
4-(2-fluoro-4-methoxyphenyl)-2-(2-methoxyphenyl)-5-(pyridin-3-ylmethyl)-1H-pyrazolo[4,3-c]pyridine-3,6(2H,5H)-dione;
2-(2-chlorophenyl)-4-(4-methoxyphenyl)-5-(pyrazin-2-ylmethyl)-1H-pyrazolo [4,3-c] pyridine-3,6(2H,5H)-dione;
4-(4-chlorophenyl)-2-(2-methoxyphenyl)-5-(pyrazin-2-ylmethyl)-1H-pyrazolo [4,3-c] pyridine-3,6(2H,5H)-dione;
2-(2-chlorophenyl)-4-(2-fluoro-4-methoxyphenyl)-5-[(1-methyl-1H-pyrazol-3-yl)methyl]-1H-pyrazolo[4,3-c]pyridine-3,6(2H,5H)-dione;
4-(2-fluoro-5-methoxyphenyl)-2-(2-methoxyphenyl)-5-(pyridin-3-ylmethyl)-1H-pyrazolo[4,3-c]pyridine-3,6(2H,5H)-dione;
2-(2-chlorophenyl)-5-[(2-methoxypyridin-4-yl)methyl]-4-methyl-1H-pyrazolo [4,3-c] pyridine-3,6(2H,5H)-dione;
2-(2-methoxyphenyl)-4-methyl-5-(pyridin-3-ylmethyl)-1H-pyrazolo[4,3-c] pyridine-3,6(2H,5H)-dione;
4-(4-chloro-2-fluorophenyl)-2-(2-methoxyphenyl)-5-(pyridin-3-ylmethyl)-1H-pyrazolo [4,3-c] pyridine-3,6(2H,5H)-dione;
4-(5-chloro-2-fluorophenyl)-2-(2-chlorophenyl)-5-(pyridin-3-ylmethyl)-1H-pyrazolo[4,3-c]pyridine-3,6(2H,5H)-dione;
2-(2-chlorophenyl)-5-[(6-methoxypyridin-3-yl)methyl]-4-methyl-1H-pyrazolo [4,3-c] pyridine-3,6 (2H,5H)-dione;
4-(4-chloro-2-fluorophenyl)-2-(2-chlorophenyl)-5-(pyridin-3-ylmethyl)-1H-pyrazolo[4,3-c]pyridine-3,6(2H,5H)-dione;
4-(5-chloro-2-fluorophenyl)-2-(2-chlorophenyl)-5-(pyridin-4-ylmethyl)-1H-pyrazolo[4,3-c]pyridine-3,6(2H,5H)-dione;
4-(2-fluoro-5-methoxyphenyl)-2-(2-methoxyphenyl)-5-[(1-methyl-1H-pyrazol-3-yl)methyl]-1H-pyrazolo[4,3-c]pyridine-3,6(2H,5H)-dione;
4-(5-chloro-2-fluorophenyl)-2-(2-methoxyphenyl)-5-(pyridin-3-ylmethyl)-1H-pyrazolo [4,3-c] pyridine-3,6(2H,5H)-dione;
2-(2-chlorophenyl)-4-methyl-5-(pyridin-3-ylmethyl)-1H-pyrazolo[4,3-c] pyridine-3,6 (2H,5H)-dione;
2-(2-chlorophenyl)-4-(4-chlorophenyl)-5-(pyrazin-2-ylmethyl)-1H-pyrazolo[4,3-c]pyridine-3,6 (2H,5H)-dione;
2-(2-chlorophenyl)-4-(2-fluorophenyl)-5-(pyridin-3-ylmethyl)-1H-pyrazolo[4,3-c] pyridine-3,6(2H,5H)-dione;
2-(2-chlorophenyl)-4-(4-chlorophenyl)-5-(pyridin-4-ylmethyl)-1H-pyrazolo[4,3-c]pyridine-3,6(2H,5H)-dione;
4-(4-chloro-2-fluorophenyl)-2-(2-chlorophenyl)-5-(pyridin-4-ylmethyl)-1H-pyrazolo[4,3-c]pyridine-3,6(2H,5H)-dione;
2-(2-methoxyphenyl)-4-(3-methoxyphenyl)-5-[(1-methyl-1H-pyrazol-3-yl) methyl]-1H-pyrazolo[4,3-c]pyridine-3,6(2H,5H)-dione;
2-(2-chlorophenyl)-4-(2-fluoro-4-methoxyphenyl)-5-(pyridin-3-ylmethyl)-1H-pyrazolo[4,3-c]pyridine-3,6(2H,5H)-dione;
4-(2-fluoro-4-methoxyphenyl)-2-(2-methoxyphenyl)-5-[(1-methyl-1H-pyrazol-3-yl)methyl]-1H-pyrazolo[4,3-c]pyridine-3,6(2H,5H)-dione;
2-(2-methoxyphenyl)-4-(4-methoxyphenyl)-5-[(1-methyl-1H-pyrazol-3-yl) methyl]-1H-pyrazolo[4,3-c]pyridine-3,6(2H,5H)-dione;
2-(2-methoxyphenyl)-4-(3-methoxyphenyl)-5-(pyridin-3-ylmethyl)-1H-pyrazolo [4,3-c] pyridine-3,6(2H,5H)-dione;
2-(2-chlorophenyl)-4-(4-chlorophenyl)-5-(pyridin-3-ylmethyl)-1H-pyrazolo[4,3-c]pyridine-3,6(2H,5H)-dione;
4-(4-chloro-2-fluorophenyl)-2-(2-chlorophenyl)-5-[(2-methoxypyridin-4-yl) methyl]-1H-pyrazolo[4,3-c]pyridine-3,6(2H,5H)-dione;
2-(2-chlorophenyl)-4-(2-fluoro-4-methoxyphenyl)-5-(pyridin-4-ylmethyl)-1H-pyrazolo[4,3-c]pyridine-3,6(2H,5H)-dione;
2-(2-chlorophenyl)-4-(2,6-difluorophenyl)-5-(pyridin-4-ylmethyl)-1H-pyrazolo [4,3-c] pyridine-3,6(2H,5H)-dione;
2-(2-chlorophenyl)-4-(2-fluorophenyl)-5-(pyridin-4-ylmethyl)-1H-pyrazolo[4,3-c]pyridine-3,6(2H,5H)-dione;
2-(2-chlorophenyl)-4-methyl-5-[(1-methyl-1H-pyrazol-3-yl)methyl]-1H-pyrazolo[4,3-c] pyridine-3,6(2H,5H)-dione;
4-(3-chloro-2-fluorophenyl)-2-(2-chlorophenyl)-5-(pyridin-4-ylmethyl)-1H-pyrazolo[4,3-c]pyridine-3,6(2H,5H)-dione;
2-(2-chlorophenyl)-5-methyl-4-[3-(methylamino)phenyl]-1H-pyrazolo[4,3-c] pyridine-3,6(2H,5H)-dione;
2-(2-methoxyphenyl)-4-(4-methoxyphenyl)-5-(pyridin-3-ylmethyl)-1H-pyrazolo [4,3-c] pyridine-3,6(2H,5H)-dione;
2-(2-chlorophenyl)-4-(2-fluorophenyl)-5-(pyridin-2-ylmethyl)-1H-pyrazolo[4,3-c]pyridine-3,6(2H,5H)-dione;
2-(2-chlorophenyl)-4-(2,5-difluorophenyl)-5-(pyridin-4-ylmethyl)-1H-pyrazolo [4,3-c]pyridine-3,6(2H,5H)-dione;
2-(2-chlorophenyl)-4-(4-chlorophenyl)-5-(1,3-thiazol-2-ylmethyl)-1H-pyrazolo [4,3-c]pyridine-3,6(2H,5H)-dione;
2-(2-chlorophenyl)-4-[3-(dimethylamino)phenyl]-5-[(1-methyl-1H-pyrazol-3-yl) methyl]-1H-pyrazolo[4,3-c]pyridine-3,6(2H,5H)-dione;
4-[3-(dimethylamino)phenyl]-2-(2-methoxyphenyl)-5-methyl-1H-pyrazolo[4,3-c]pyridine-3,6(2H,5H)-dione;
2-(2-chlorophenyl)-4-(3,5-dichlorophenyl)-5-(pyridin-4-ylmethyl)-1H-pyrazolo [4,3-c]pyridine-3,6(2H,5H)-dione;
4-(3-chloro-2-fluorophenyl)-2-(2-chlorophenyl)-5-(pyridin-3-ylmethyl)-1H-pyrazolo [4,3-c]pyridine-3,6(2H,5H)-dione;
2-(2-chlorophenyl)-4-[3-(dimethylamino)phenyl]-5-(pyridin-3-ylmethyl)-1H-pyrazolo [4,3-c]pyridine-3,6(2H,5H)-dione;
2-(2-chlorophenyl)-4-(2,6-difluorophenyl)-5-(pyridin-3-ylmethyl)-1H-pyrazolo [4,3-c] pyridine-3,6(2H,5H)-dione;
4-(2-fluoro-5-methoxyphenyl)-2-(2-methoxyphenyl)-5-(pyrazin-2-ylmethyl)-1H-pyrazolo[4,3-c]pyridine-3,6(2H,5H)-dione; and
2-(2-chlorophenyl)-4-(2,5-difluorophenyl)-5-(pyridin-3-ylmethyl)-1H-pyrazolo [4,3-c] pyridine-3,6(2H,5H)-dione, as well as tautomers, geometrical isomers, optically active forms and pharmaceutically acceptable salts thereof.

7. A Nox4 inhibitor according to claim 6 for use as a medicament.

8. A pharmaceutical composition containing at least one Nox4 inhibitor according to claim 6, and a pharmaceutically acceptable carrier, diluent or excipient thereof.

9. A Nox4 inhibitor according to any one of claims 1 to 5 wherein the Nox4 inhibitor is according to claim 6.

10. A Nox4 inhibitor according to any one of claims 1 to 5 or 9 wherein the Nox4 inhibitor is to be administered with at least one co-agent useful in the treatment or prevention of osteoporosis.

11. A Nox4 inhibitor according to claim 10 wherein the co-agent is selected from bisphosphonate, estrogen and vitamin D.

12. A pharmaceutical composition containing at least one Nox4 inhibitor combined with at least one co-agent useful in the prevention and/or treatment of an osteoclastogenesis dysfunction and/or osteoporosis, and at least one pharmaceutically acceptable carrier.

13. A pharmaceutical composition according to claim 12 wherein the Nox4 inhibitor is according to claim 6.

14. A pharmaceutical composition according to claims 12 or 13 wherein the co-agent is selected from bisphosphonate, estrogen and vitamin D.

15. A method of identifying an inhibitor of ostoeclastogenesis comprising the following steps:
(i) Contacting a substance to be screened with an encoding or expressing system for Nox4;
(ii) Assessing Nox4 activity or Nox-4 expression ability of the said system;
(iii) Comparing the Nox4 activity or Nox4 expression ability in step (ii) with the Nox4 activity or Nox4expression ability of the system in the absence of the substance;
(iv) Selecting a substance for which a decrease in Nox4 activity or Nox4 expression ability in step (iii) is observed.
